# EUROPÄISCHE PATENTSCHRIFT

(11) **EP 1 912 620 B1**
(45) Veröffentlichungstag und Bekanntmachung des Hinweises auf die Patenterteilung: **20.02.2013**
(21) Anmeldenummer: 06764243.9
(22) Anmeldetag: 21.07.2006
(51) Int. Cl.: A61K 8/81, C08F 220/04, C08F 220/34, C08F 226/00

(54) **VERWENDUNG ANIONISCH UND KATIONISCH AMPHOLYTISCHER COPOLYMERE**
USE OF ANIONICALLY AND CATIONICALLY AMPHOLYTIC COPOLYMERS
UTILISATION DE COPOLYMERES AMPHOLYTES ANIONIQUES ET CATIONIQUES

(30) Priorität: 22.07.2005 DE 102005034412; 26.07.2005 DE 102005034906; 30.09.2005 DE 102005046916; 30.09.2005 DE 102005046918
(43) Veröffentlichungstag der Anmeldung: 23.04.2008
(73) Patentinhaber: BASF SE, 67056 Ludwigshafen (DE)
(72) Erfinder: NGUYEN-KIM, Son, 69502 Hemsbach (DE); LAUBENDER, Matthias, 67105 Schifferstadt (DE); HÖSSEL, Peter, 67105 Schifferstadt (DE)
(86) Internationale Anmeldenummer: PCT/EP2006/064506
(87) Internationale Veröffentlichungsnummer: WO 2007/010035

(56) Entgegenhaltungen:
- EP-A2- 0 524 557
- WO-A-00/39176
- WO-A-01/62809
- WO-A-93/22358
- WO-A2-2004/058837
- WO-A2-2006/044193

## Beschreibung

Die folgende Erfindung betrifft die Verwendung anionisch ampholytischer und kationisch ampholytischer Copolymere als Rheotogiemodifizierungsmittel für haarkosmetische Zusammensetzungen.

An haarkosmetische Zusammensetzungen werden häufig spezielle Anforderungen bezüglich ihrer rheologischen Eigenschaften gestellt. Häufig können sie nur mit Hilfe von Zusatzstoffen, so genannten Verdickem", in die gewünschte Anwendungsform gebracht werden. Beispiele für übliche niedermolekulare Verdickungsmittel sind z. B. die Alkali- und Aluminiumsalze von Fettsäuren, Fettalkohole oder Wachse. Die Verwendung der bekannten Verdickungsmittel ist jedoch, je nach Einsatzgebiet der zu verdickenden Zubereitung, häufig mit Nachteilen verbunden. So kann entweder die Verdickungswirkung der Verdickungsmittel nicht zufrieden stellend, ihr Einsatz unerwünscht oder ihre Einarbeitung in die zu verdickende Zubereitung, beispielsweise wegen ihrer Unverträglichkeit mit der zu verdickenden Verbindung erschwert oder ganz unmöglich sein. Schwierigkeiten bereitet oft die Bereitstellung von Produkten mit einem komplexen Eigenschaftsprofil unter Einsatz eines möglichst geringen Anteils oder möglichst wenig verschiedener wirkaktiver Substanzen. So besteht beispielsweise ein Bedarf an Polymeren für haarkosmetische Mittel, die eine Einstellung der rheologischen Eigenschaften der Mittel ermöglichen und die zusätzlich über gute filmbildende Eigenschaften verfügen. Zudem werden an haarkosmetische Produkte vom Verbraucher zunehmend ästhetische Anforderungen gestellt. So wird bei derartigen Produkten derzeit eine Bevorzugung von klaren, opaken Formulieren in Form von Gelen beobachtet. Eine weitere vorteilhafte Eigenschaft von Polymeren für haarkosmetische Formulierungen ist die Befähigung sich in eine feste Form, insbesondere ein Pulver überführen zu lassen und dennoch befähigt sein, sich in kurzer Zeit in eine zu verdickende Zusammensetzung einarbeiten zu lassen und dabei zuverlässig die gewünschten Rheologieeigenschaften bereit zu stellen.

Die WO 01/85821 beschreibt Polyurethane und deren Verwendung zur Modifizierung rheologischer Eigenschaften.

Die US 3,915,921 beschreibt Copolymere, die eine olefinisch ungesättigte Carbonsäure, ein C₁₀-C₃₀-Alkyl(meth)acrylat und gegebenenfalls ein vernetzendes Monomer mit wenigstens zwei ethylenisch ungesättigten Doppelbindungen einpolymerisiert enthalten. In neutralisierter Form dienen sie als Verdicker für diverse Anwendungen.

Die WO 97/21744 beschreibt vernetzte anionische Copolymere und deren Verwendung als Verdickungs- und Dispergiermittel in wässrigen Systemen.

Die EP-A-0 982 021 beschreibt die Verwendung von (teil)neutralisierten Copolymerisaten aus
A) 50 bis 99 Gew.-% monoethylenisch ungesättigten Carbonsäuren und
B) 1 bis 50 Gew.-% mindestens eines Comonomers, ausgewählt unter
   a) monoethylenisch ungesättigten Carbonsäureestern mit gesättigten C₈-C₃₀-Alkoholen,
   b) N-C₈-C₁₈-Alkyl- und N,N-Di-C₈-C₁₈-alkylcarbonsäureamiden,
   c) Vinylestern aliphatischer C₈-C₃₀-Carbonsäuren,
   d) C₈-C₁₈-Alkylvinylethem,
und Mischungen davon
als Verdicker zur Herstellung von Haarwaschmitteln.

In der US 4,395,524 und der US 4,432,881 werden als Verdicker wirksame Copolymere auf Basis von Amidgruppen-haltigen Monomeren beschrieben.

Die DE-A-42 13 971 beschreibt Copolymere, die wenigstens ein olefinisch ungesättigtes Säuregruppen-haltiges Monomer, wenigstens eine olefinisch ungesättigte quartäre Ammoniumverbindung, gegebenenfalls wenigstens ein Polyether(meth)acrylat und gegebenenfalls wenigstens einen Vemetzer einpolymerisiert enthalten und deren Verwendung als Verdickungsmittel zur Verdickung wässriger Systeme, wobei es sich um kosmetische Zubereitungen handeln kann.

Die EP-A-893 117 und die EP-A-913 143 beschreiben vernetzte kationische Copolymere und deren Verwendung u. a. als haarfestigende Gelbildner in kosmetischen Zusammensetzungen.

Die EP-A-1 064 924 beschreibt die Verwendung von vernetzten kationischen Polymeren in hautkosmetischen und dermatologischen Zubereitungen, u. a. als Verdicker.

Die US 5,015,708 beschreibt ein Verfahren zur Herstellung eines Terpolymers aus (i) einem Vinyllactam, (ii) einem Säuregruppen-haltigen Monomer und (iii) einem hydrophoben Monomer, wobei es sich u. a. um eine ethylenisch ungesättigte Siliconverbindung handeln kann, durch Fällungspolymerisation sowie die Herstellung von Pulvern aus diesen Polymeren.

Die WO 01/62809 beschreibt ein kosmetisches Mittel; das wenigstens ein wasserlösliches oder wasserdispergierbares Polymer enthält, das
a) 5 bis 50 Gew.-% wenigstens eines α,β-ethylenisch ungesättigten Monomers mit einer tert.-Butylgruppe,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül, und
d) 0 bis 30 Gew.-% wenigstens einer weiteren α,β-ethylenisch ungesättigten Verbindung, wobei es sich um Verbindungen mit mindestens einer anionogenen und/oder anionischen Gruppe pro Molekül handeln kann,
eingebaut enthält.

Die EP-A-670 333 beschreibt vernetzte wasserlösliche Polymerdispersionen, die durch Polymerisation eines Monomergemischs, enthaltend wenigstens ein wasserlösliches Monomer, wenigstens einen Vernetzer sowie gegebenenfalls hydrophobe und/oder amphiphile Monomere in Gegenwart eines polymeren Dispergiermittels erhältlich sind. Als wasserlösliche Monomere können neben einer Vielzahl weiterer auch N-Vinylpyrrolidon sowie Monomere mit kationischen/kationisierbaren Gruppen, wie N-Vinylimidazol, eingesetzt werden. Bei den polymeren Dispergiermitteln kann es sich um Polyelektrolyte handeln, die beispielsweise Salze der (Meth)acrylsäure als anionische Monomerbausteine oder quaternierte Derivate von N,N-Dimethylaminoethyl-(meth)acrylat als kationische Bausteine einpolymerisiert enthalten. Ein Einsatz der Polymerdispersionen in der Kosmetik wird nicht beschrieben.

Die EP-A-929 285 lehrt die Verwendung von wasserlöslichen Copolymeren, die Vinylcarbonsäureamid-Einheiten und Vinylimidazol-Einheiten einpolymerisiert enthalten als Bestandteil kosmetischer Mittel. Polyelektrolyt-Komplexe dieser Copolymere mit Carbonsäuregruppen-haltigen Polymeren sind in diesem Dokument nicht offenbart.

Die WO 00/27893 beschreibt wässrige Polymerdispersionen auf der Basis von N-Vinylcarbonsäureamiden und gegebenenfalls Comonomeren, wobei neben einer großen Anzahl weiterer auch N-Vinylpyrrolidon, N-Vinylimidazol und N-Vinylimidazolderivate genannt werden. Die Polymerisation erfolgt in Gegenwart wenigstens eines polymeren Dispergiermittels. Ein Einsatz dieser Polymerdispersionen in der Kosmetik wird nur ganz allgemein und ohne Beleg durch ein Ausführungsbeispiel beschrieben.

Die EP-A-1038891 beschreibt wasserlösliche oder wasserdispergierbare polymere Salze aus wenigstens einem Polymer und wenigstens einem entgegengesetzt geladenen Neutralisationsmittel.

Die WO 02/41856 beschreibt die Verwendung von Polymerdispersionen, die durch Polymerisation wenigstens eines wasserlöslichen Monomers in einer wässrigen Salzlösung, die wenigstens einen Polyelektrolyten als Dispergiermittel enthält, erhältlich sind zur kosmetischen Behandlung von keratinischen Materialien. Zusätzlich enthalten die Dispersionen wenigstens ein Mittel zur Einstellung der Viskosität, beispielsweise eine Polycarbonsäure oder ein Salz davon. Als wasserlösliche Monomere können kationische, anionische und nichtionische Monomere eingesetzt werden, bevorzugt sind Monomergemische, die wenigstens ein kationisches Monomer sowie gegebenenfalls zusätzlich Acrylsäure und/oder Acrylamid enthalten.

Die WO 2005/004821 beschreibt ein kosmetisches oder pharmazeutisches Mittel, das wenigstens einen Polyelektrolyt-Komplex enthält, der wenigstens ein wasserlösliches oder wasserdispergierbares Copolymer A1) mit kationogenen Gruppen, das Vinylimidazol und/oder ein Derivat davon und wenigstens ein weiteres damit copolymerisierbares Monomer einpolymerisiert enthält, und wenigstens ein Säuregruppen-haltiges Polymer A2) umfasst.

Die WO 2005/005497 beschreibt eine wässrige Polymerdispersion Pd), die erhältlich ist durch radikalische Polymerisation eines Monomergemischs M), enthaltend
a) wenigstens eine α,β-ethylenisch ungesättigte amidgruppenhaltige Verbindung der allgemeinen Formel I 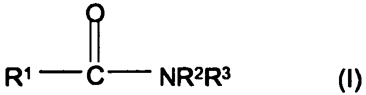 wobei
   R² für eine Gruppe der Formel CH₂=CR⁴- steht und R¹ und R³ unabhängig voneinander für H, Alkyl, Cycloalkyl, Heterocycloalkyl, Aryl oder Hetaryl stehen, oder R¹ und R³ gemeinsam mit der Amidgruppe, an die sie gebunden sind, für ein Lactam mit 5 bis 8 Ringatomen stehen,
b) wenigstens eine radikalisch polymerisierbare vernetzende Verbindung mit wenigstens zwei α,β-ethylenisch ungesättigten Doppelbindungen pro Molekül,
c) wenigstens eine Verbindung mit einer radikalisch polymerisierbaren α,β-ethylenisch ungesättigten Doppelbindung und mindestens einer kationogenen und/oder kationischen Gruppe pro Molekül,
in einem wässrigen Medium in Gegenwart wenigstens eines polymeren anionischen Dispergiermittels D). Beschrieben sind weiterhin kosmetische oder pharmazeutische Mittel, die eine solche Polymerdispersion oder ein durch Trocknen einer solchen Dispersion erhältliches Polymer enthalten.

Die WO 00/39176 beschreibt ein hydrophiles, kationisches, ampholytisches Copolymer, das 0,05 bis 20 Mol-% eines anionischen Monomers mit wenigstens einer Carboxygruppe, 0 bis 45 Mol-% eines kationischen Monomers mit wenigstens einer Aminogruppe sowie gegebenenfalls ein hydrophobes Monomer und/oder einen Vemetzer einpolymerisiert enthält, wobei das Molverhältnis von kationischem zu anionischem Monomer etwa 2 : 1 bis 16 : 1 beträgt.

Die WO 04/058837 beschreibt ein ampholytisches Copolymer, das durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung
sowie gegebenenfalls weiterer Comonomere erhältlich ist. Die Polymerisation kann in Gegenwart einer Pfropfgrundlage erfolgen, wobei es sich u. a. um ein Polyalkylenoxidhaltiges Siliconderivat handeln kann. Beschrieben sind weiterhin Polyelektrolyt-Komplexe, die ein solches ampholytisches Copolymer enthalten sowie kosmetische oder pharmazeutische Mittel auf Basis dieser Silicongruppen-haltigen Copolymere und Polyelektrolyt-Komplexe.

Die US 2006/0084586 A1 (= WO 2006/0441931) beschreibt Rheologie-modifizierende Haarfestigerharze, die ein vernetztes Copolymer auf Basis von Vinylamid- und Carbonsäuremonomeren enthalten. Polymere, die durch radikalische Polymerisation in Gegenwart wenigstens einer eine Polyethergruppe und/oder eine radikalisch polymerisierbare Doppelbindung aufweisenden Siliconverbindung erhältlich sind, sind nicht beschrieben. Polymere, die wenigstens eine kationische Verbindung, die ausgewählt ist unter N-VinylimidazolVerbindungen, N-[3-(dimethylamino)propyl]acrylamid, N-[3-(dimethylamino)propyl]methacrylamid und Mischungen davon einpolymerisiert enthalten, sind ebenfalls nicht beschrieben.

Die EP 0 524 557 A2 beschreibt eine Kontaktlinse, die ein Copolymer enthält, das als Hauptkomponenten A) 15 - 40 Mol-% eines Fluoralkyl/Si-alkyl-fumarats, B) 35 - 55 Mol-% eines N-Vinyllactams, C) 3 - 30 Mol-% (Meth)acrylsäure und D) 0,1 - 20 Mol-% eines Vernetzers einpolymerisiert enthält. Die WO 93/22358 beschreibt Copolymerisate aus Carbonsäuren und quatenrären Ammoniumverbindungen und ihre Verwendung als Verdickungs- oder Dispergiermittel.

Es besteht weiterhin ein Bedarf an Verdickern für haarkosmetische Zusammensetzungen mit verbesserten Eigenschaften. So weisen die derzeit für diese Anwendungen noch am häufigsten eingesetzten anionischen Verdicker auf Basis von Acrylsäure, wie die entsprechenden Carbopole, eine Reihe von Nachteilen auf. Sie sind selbst nicht zur Filmbildung befähigt, so dass zur Herstellung von Mitteln mit festigender Wirkung immer der Einsatz größerer Mengen filmbildender Polymere erforderlich ist. Da anionische Verdicker auf Basis von Acrylsäure jedoch mit einer Vielzahl von Polyelektrolyten unverträglich sind, kommen als Formulierungspartner überwiegend nur nichtionische Polymere in Frage. Damit lässt sich eine Vielzahl von Produkten mit interessanten Anwendungseigenschaften, wie z. B. Gele auf Basis kationischer Polymere, überhaupt nicht herstellen.

Es besteht insbesondere ein Bedarf an polymeren Verdickern für haarkosmetische Zusammensetzungen, die sich zur Formulierung von gelförmigen Präparaten eignen. Diese sollen möglichst viele der folgenden Eigenschaften in sich vereinen:
- die erhaltenen Gele sollen möglichst klar sein,
- sie sollen sich leicht im Haar verteilen lassen und diesem dennoch guten Halt verleihen, was sich besonders gut durch Gele mit thixotropen Eigenschaften erzielen lässt,
- sie sollen selbst über filmbildende Eigenschaften verfügen und so zur Festigung der Haare beitragen,
- sie sollen über konditionierende Eigenschaften verfügen und die sensorischen Eigenschaften des Haars verbessern, z. B. ihm Geschmeidigkeit und Glanz verleihen und nach dem Trocken nicht oder nur gering klebrig sein,
- das mit dem Gel behandelte Haar soll eine gute Nasskämmbarkeit aufweisen (somit lässt sich das frisch behandelte Haar leicht mit dem Kamm in Form bringen, um die gewünschte Frisur zu formen),
- Befähigung zur Formulierung von Gelen in möglichst allen kosmetisch akzeptablen pH-Bereichen, speziell in einem pH-Bereich von etwa 3 bis 9,
- die Befähigung zur Formulierung von Gelen mit gezielt über den pH-Wert schaltbaren Eigenschaften.

Zudem sollen sich Polymere zur Verwendung als Rheologiemodifizierungsmittel für kosmetische Zusammensetzungen zur Behandlung der Haare leicht in eine feste Form, vorzugsweise ein Pulver, überführen lassen, das sich leicht in die zu verdickenden Formulierungen einarbeiten lässt.

Erfindungsgemäß wird diese Aufgabe gelöst durch die Verwendung eines ampholytischen Copolymers, erhältlich durch radikalische Copolymerisation nach der Methode der Fällungspolymerisation, das einen molaren Überschuss an anionogenen/anionischen Gruppen gegenüber kationogenen/kationischen Gruppen aufweist oder das einen molaren Überschuss an kationogenen/kationischen Gruppen gegenüber anionogenen/anionischen Gruppen aufweist und das Verbindungen, bestehend aus
a1) 2 bis 96 Gew.-% Acrylsäure und/oder Methacrylsäure,
a2) 2 bis 96 Gew.-% wenigstens einer N-Vinylimidazol-Verbindung der allgemeinen Formel (II) 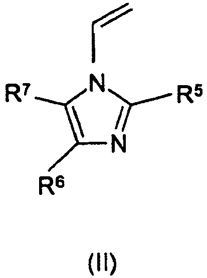 worin R⁵ bis R⁷ unabhängig voneinander für Wasserstoff, C₁-C₄-Alkyl oder Phenyl stehen, oder die durch Protonierung oder Quaternisierung der N-Vinglimidazol-Verbindugen erhältlichen Verbindungen,
   b) 0,05 bis 5 Gew.-% Ethylenglykoldi(meth)acrylat und/oder Pentaerythrittriallylether,
   c) 0 bis 15 Gew.% wenigstens einer Siliconverbindung,
   d) 0 bis 95 Gew.-% Vinylpyrrolidon und/oder Vinylcaprolactam,
   e) 0 bis 40 Gew.-% wenigstens einer Verbindung, die ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
   f) 0 bis 40 Gew.-% wenigstens eines Monomers, das ausgewählt ist unter Methyl(meth)acrylat, Ethyl(meth)acrylat, n-Butyl(meth)acrylat und Mischungen davon,
      einpolymerisiert enthält,
      wobei das ampholytische Copolymer einen molaren Überschuss an anionogenen/anionischen Gruppen gegenüber kationogenen/kationischen Gruppen oder einen molaren Überschuss an kationogenen/kationischen Gruppen gegenüber anionogenen/anionischen Gruppen von wenigstens 2,5 : 1 aufweist,
      als Rheologiemodifizierungsmittel für haarkosmetische Zusammensetzungen.

Ein weiterer Gegenstand der Erfindung ist ein Verfahren zur kosmetischen Behandlung der Haare, bei dem man ein haarkosmetisches Mittel, enthaltend
A) wenigstens ein ampholytisches Copolymer, wie zuvor und im Folgenden definiert,
B) gegebenenfalls wenigstens ein von A) verschiedenes Haarpolymer,
C) wenigstens einen kosmetisch akzeptablen Träger, und
D) gegebenenfalls wenigstens einen von A) und B) verschiedenen kosmetisch akzeptablen Wirk- und/oder Hilfsstoff,
bereitstellt und auf die zu behandelnden Haare appliziert.

Copolymere, die einen molaren Überschuss an anionogenen/anionischen Gruppen gegenüber kationogenen/kationischen Gruppen aufweisen, werden im Rahmen der

Erfindung auch als anionische ampholytische Copolymere bezeichnet Copolymere, die einen molaren Überschuss an kationogenen/kationischen Gruppen gegenüber anionogenen/anionischen Gruppen aufweisen, werden im Rahmen der Erfindung auch als kationische ampholytische Copolymere bezeichnet.

In einer ersten Ausführungsform werden für die erfindungsgemäße Verwendung Silicongruppen-freie Copolymere eingesetzt. Dafür eignen sich alle in der deutschen Patentanmeldung 10 2005 034 412.7 beschriebenen Copolymere, die einen molaren Überschuss an anionogenen/anionischen Gruppen gegenüber kationogenen/kationischen Gruppen oder die einen molaren Überschuss an kationogenen/kationischen Gruppen gegenüber anionogenen/anionischen Gruppen aufweisen. Auf die Offenbarung dieses Dokuments wird bezüglich dieser Copolymere in vollem Umfang Bezug genommen.

In einer zweiten Ausführungsform werden für die erfindungsgemäße Verwendung Silicongruppen-haltige Copolymere eingesetzt. Dafür eignen sich alle in der deutschen Patentanmeldung 10 2005 034 906.4 beschriebenen Copolymere, die einen molaren Überschuss an anionogenen/anionischen Gruppen gegenüber kationogenen/kationischen Gruppen oder die einen molaren Überschuss an kationogenen/kationischen Gruppen gegenüber anionogenen/anionischen Gruppen aufweisen. Auf die Offenbarung dieses Dokuments wird bezüglich dieser Copolymere in vollem Umfang Bezug genommen.

Die Verwendung eines ampholytischen Copolymers, das einen molaren Überschuss an anionogenen/anionischen Gruppen aufweist, ist in der deutschen Prioritätsanmeldung 10 2005 046 918.3 beschrieben. Die Verwendung eines ampholytischen Copolymers, das einen molaren Überschuss an kationogenen/kationischen Gruppen aufweist, ist in der deutschen Prioritätsanmeldung 10 2005 046 916.7 beschrieben. Auf diese Offenbarung wird in vollem Umfang Bezug genommen.

Die Herstellung der erfindungsgemäß zu verwendenden Copolymere A) erfolgt durch radikalische Copolymerisation nach der Methode der Fällungspolymerisation in wenigstens einem organischen Lösungsmittel. In einer speziellen Ausführung werden zur Herstellung der erfindungsgemäß eingesetzten Copolymere wenigstens zwei Radikalinitiatoren eingesetzt, deren Zerfallstemperaturen und/oder deren Halbwertszeiten bei einer bestimmten Polymerisationstemperatur voneinander verschieden sind. Dabei können Copolymere mit besonders geringen Restmonomergehalten erzielt werden. Dies ist insbesondere der Fall, wenn der bei höherer Temperatur zerfallende Initiator vor Abschluss, vorzugsweise vor Beginn der Fällung des Polymers, zugegeben wird.

Bei der Fällungspolymerisation sind die eingesetzten Monomere im Reäktionsmedium (Monomer, Lösungsmittel) löslich, das entsprechende Polymer aber nicht. Das entstehende Polymer wird unter den gewählten Polymerisationsbedingungen unlöslich und fällt aus dem Reaktionsgemisch aus. Dabei lassen sich anionogene/anionische ampholytische Copolymere mit höheren Molekulargewichten erhalten, als nach anderen Polymerisationsverfahren, z. B. durch Lösungspolymerisation, die sich besonders vorteilhaft als Rheologiemodifizierer (speziell Verdicker) eignen.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Dazu zählen z. B. n-Hexyl(en), n-Heptyl(en), n-Octyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en), Arachinyl(en), Behenyl(en), Lignocerinyl(en), Melissinyl(en), etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäß verwendeten Copolymere lassen sich unter Normalbedingungen (20 °C) vorteilhaft als Gele formulieren. "Gelförmige Konsistenz" zeigen Formulierungen, die eine höhere Viskosität als eine Flüssigkeit aufweisen und die selbsttragend sind, d. h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren Die Viskosität der gelförmigen Mittel liegt vorzugsweise in einem Bereich von größer als 600 bis etwa 60000 mPas, besonders bevorzugt von 6000 bis 30000 mPas. Vorzugsweise handelt es sich bei den Gelen um Haargele.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäß verwendeten Copolymere sind im Allgemeinen wasserlöslich.

"Modifizierung rheologischer Eigenschaften" wird im Rahmen der vorliegenden Erfindung weit verstanden. So eignen sich die erfindungsgemäß verwendeten Copolymere im Allgemeinen zur Verdickung der Konsistenz von flüssigen Verbindungen in einem weiten Bereich. Je nach Grundkonsistenz der flüssigen Verbindung(en) können in Abhängigkeit von der Einsatzmenge des Copolymers in der Regel Fließeigenschaften von dünnflüssig bis hin zu fest (nicht mehr fließend) erzielt werden. Unter "Modifizierung rheologischer Eigenschaften" wird daher u. a. die Erhöhung der Viskosität von Flüssigkeiten, die Verbesserung der Thixotropie-Eigenschaften von Gelen, die Verfestigung von Gelen und Wachsen etc. verstanden.

Die erfindungsgemäß verwendeten ampholytischen Copolymere weisen sowohl anionogene und/oder anionische Gruppen als auch kationogene und/oder kationische Gruppen auf. Zu ihrer Herstellung können die entgegengesetzt geladenen/ladbaren Monomere a1) und a2) gemeinsam, d. h. in Form eines Monomerenpaares ("Monomerensalzes") eingesetzt werden. In dieser Monomerzusammensetzung beträgt das molare Verhältnis von anionogenen und anionischen Gruppen der Komponente a1) zu kationogenen und kationischen Gruppen der Komponente a2) 1 : 1 (d. h. einwertige Monomere werden im Wesentlichen äquimolar eingesetzt). Die Monomerenpaare können dabei vor ihrem Einsatz zur Polymerisation separat hergestellt werden. Bevorzugt ist jedoch die "in situ"-Herstellung der Monomerenpaare durch gemeinsamen Einsatz (z. B. gemeinsamen Zulauf) bei der Herstellung der Copolymerisate.

Vorzugsweise beträgt der Anteil des Monomerpaares an den zur Polymerisation eingesetzten Verbindungen wenigstens 1 Gew.-%, bevorzugt wenigstens 2 Gew.-%, insbesondere wenigstens 3 Gew.-%.

Die Copolymere weisen einen deutlichen Überschuss an anionogenen/anionischen Gruppen oder an kationogenen/kationischen Gruppen auf. Erfindungsgemäβ werden zur Copolymerisation Monomere mit ionogenen oder ionischen Gruppen in solchen Mengen eingesetzt werden, dass das Copolymer einen molaren Überschuss an anionogenen/anionischen Gruppen gegenüber kationogenen/kationischen oder einen molaren Überschuss an kationogenen/kationischen Gruppen gegenüber anionogenen/anionischen Gruppen von wenigstens 2,5 : 1, aufweist.

### Monomer a1)

Die Komponente a1) wird bei anionisch ampholytischen Copolymeren vorzugsweise in einer Menge von 2 bis 70 Gew.-%, vorzugsweise 3 bis 60 Gew.-%, bezogen auf das Gesamtgewicht der zur Polymerisation eingesetzten Verbindungen (d. h. Komponenten a1), a2), b), c) und, falls vorhanden, d) bis f), eingesetzt.

Erfindungsgemäβ ist die Komponente a1) ausgewählt unter Acrylsäure, Methacrylsäure und Mischungen davon. In einer speziellen Ausführung umfasst die Komponente a1) Methacrylsäure oder besteht daraus. In einer weiteren speziellen Ausführung umfasst die Komponente a1) Acrylsäure oder besteht daraus.

### Monomer a2)

Die Komponente a2) kann im Allgemeinen in einer Menge von 2 bis 90 Gew.-%, bezogen auf das Gesamtgewicht der zur Polymerisation eingesetzten Verbindungen, eingesetzt werden. Die anionisch ampholytischen Copolymere enthalten vorzugsweise 2 bis 35 Gew.-%, besonders bevorzugt 3 bis 30 Gew.-%, bezogen auf das Gesamtgewicht der zur Polymerisation eingesetzten Monomere, wenigstens eines Monomers a2) einpolymerisiert. Die kationisch ampholytischen Copolymere enthalten vorzugsweise 2 bis 90 Gew.-%, besonders bevorzugt 3 bis 70 Gew.-%, insbesondere 5 bis 70 Gew.-%, bezogen auf das Gesamtgewicht der zur Polymerisation eingesetzten Monomere, wenigstens eines Monomers a2) einpolymerisiert.

Geladene kationische Gruppen lassen sich aus den Aminstickstoffen entweder durch Protonierung oder durch Quatemisierung mit Säuren oder Alkylierungsmitteln erzeugen. Dazu zählen z. B. Carbonsäuren, wie Milchsäure, Weinsäure oder Citronensäure, oder Mineralsäuren, wie Phosphorsäure, Schwefelsäure und Salzsäure, oder als Alkylierungsmittel C₁-C₄-Alkylhalogenide oder -sulfate, wie Ethylchlorid, Ethylbromid, Methylchlorid, Methylbromid, Dimethylsulfat und Diethylsulfat. Eine Protonierung oder Quatemisierung kann im Allgemeinen sowohl vor als auch nach der Polymerisation erfolgen, vorzugsweise nach der Polymerisation.

Erfindungsgemäβ enthlält das Copolymer als Monomer a2) wenigstens eine N-Vinylimidazol-Verbindung der allgemeinen Formel (II) 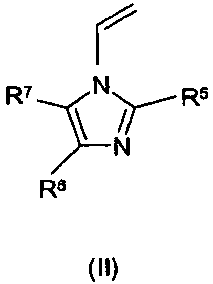 einpolymerisiert, worin R⁵ bis R⁷ unabhängig voneinander für Wasserstoff, C₁-C₄-Alkyl oder Phenyl stehen.

Beispiele für Verbindungen der allgemeinen Formel (II) sind folgender Tabelle 1 zu entnehmen:

**Tabelle 1**

| **R⁵** | **R⁶** | **R⁷** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer a2) ist 1-Vinylimidazol (N-Vinylimidazol) und sind Mischungen, die N-Vinylimidazol enthalten.

Geeignete Monomere a2) sind auch die durch Protonierung oder Quaternisierung der zuvor genannten N-Vinylimidazolverbindungen erhältlichen Verbindungen. Beispiele für solche geladenen Monomere a2) sind quatemisierte Vinylimidazole, insbesondere 3-Methyl-1-vinylimidazoliumchlorid und -methosulfat. Geeignete Säuren und Alkylierungsmittel sind die zuvor aufgeführten.

Wird wenigstens eine N-Vinylimidazol-Verbindung, speziell N-Vinylimidazol, als alleiniges Monomer a2) eingesetzt, so beträgt der Anteil vorzugsweise 3 bis 96 Gew.-%, bezogen auf das Gesamtgewicht der zur Polymerisation eingesetzten Verbindungen.

### Vemetzer b)

Die Copolymere enthalten wenigstens einen Vernetzer, d. h. eine Verbindung mit zwei oder mehr als zwei ethylenisch ungesättigten, nichtkonjugierten Doppelbindungen einpolymerisiert.

Vorzugsweise werden Vemetzer in einer Menge von 0,1 bis 4 Gew.-%, bezogen auf das Gesamtgewicht der zur Polymerisation eingesetzten Monomere, verwendet.

Enfindungsgemäβe Vernetzer b) sind Ethylenglykoldi(meth)acrylat und/oder Pentaerythrittriallylether.

### Siliconverbindung c)

In der eingangs beschriebenen ersten Ausführungsform werden Silicongruppen-freie Copolymere eingesetzt. Dafür eignen sich, wie bereits erwähnt, alle in der deutschen Patentanmeldung 10 2005 034 412.7 beschriebenen Copolymere. In der eingangs beschriebenen zweiten Ausführungsform der Erfindung werden Silicongruppen-haltige Copolymere eingesetzt. Wie bereits ausgeführt, eignen sich dafür alle in der deutschen Patentanmeldung 10 2005 034 906.4 beschriebenen Copolymere, die einen molaren Überschuss an anionogenenlanionischen Gruppen gegenüber kationogenenlkationischen Gruppen aufweisen.

Die erfindungsgemäß verwendeten Silicongruppen-haltigen Copolymere werden durch Polymerisation gemeinsam mit wenigstens einer Siliconverbindung hergestellt. Die Einsatzmenge der Siliconverbindung c) beträgt vorzugsweise 0,05 bis 30 Gew.-%, besonders bevorzugt 0,1 bis 20 Gew.-%, insbesondere 0,2 bis 10 Gew.-%, speziell 0,5 bis 5 Gew.-%, bezogen auf das Gesamtgewicht der zur Polymerisation eingesetzten Verbindungen.

Geeignete Siliconverbindungen c) sind sowohl Verbindungen, die eine radikalisch polymerisierbare olefinisch ungesättigte Doppelbindung aufweisen, als auch Verbindungen, die an Stelle einer solchen Doppelbindung eine Polyethergruppe aufweisen. Geeignet sich selbstverständlich auch Verbindungen, die sowohl wenigstens eine radikalisch polymerisierbare Doppelbindung als auch eine Polyethergruppe aufweisen.

In einer ersten Ausführungsform erfolgt die radikalische Copolymerisation zur Herstellung der Silicongruppen-haltigen Copolymere in Gegenwart wenigstens eine Polyetherhaltigen Siliconverbindung c), die keine α,β-ethylenisch ungesättigte Doppelbindung enthält. Die Copolymerisation erfolgt dann insbesondere nach der Methode der Fällungspolymerisation in wenigstens einem organischen Lösungsmittel. Die Polymerisationstemperatur liegt dann bevorzugt bei wenigstens 70 °C, insbesondere bevorzugt wenigstens 80 °C. Bei der radikalischen Copolymerisation in Gegenwart einer solchen Komponente c) werden Copolymere mit vorteilhaften Eigenschaften erhalten. Dies kann beispielsweise auf eine Wirkung der Komponente c) als Schutzkolloid oder Emulgator zurückzuführen sein. Dies kann beispielsweise auch aus einer zumindest teilweisen Pfropfung auf die Komponente c) als Pfropfgrundlage resultieren. Es sind jedoch auch andere Mechanismen als eine Pfropfung vorstellbar. Die erfindungsgemäß verwendeten Silicongruppen-haltigen Copolymere umfassen ganz allgemein die Verfahrensprodukte der radikalischen Copolymerisation, worunter z. B. reine Pfropfpolymerisate, Mischungen von Pfropfpolymerisaten mit ungepfropften Verbindungen der Komponente c), Copolymerisate der zuvor genannten Monomere sowie beliebige Mischungen verstanden werden.

Geeignete Silikonderivate c) sind die unter den INCI-Namen Dimethicone-Copolyole oder Silicontenside bekannten Verbindungen, wie zum Beispiel die unter den Markennamen Abil® (der Fa. Th. Goldschmidt), Alkasil® (der Fa. Rhône-Poulenc), Silicone Polyol Copolymer® (der Fa Genesee), Belsil® (der Fa. Wacker), Silwet® (der Fa. OSI) oder Dow Coming (der Fa. Dow Coming) erhältlich Verbindungen. Diese beinhalten Verbindungen mit den CAS-Nummem 64365-23-7; 68937-54-2; 68938-54-5; 68937-55-3. Eine geeignete, kommerziell erhältliche Verbindung ist Belsil ® DMC 6031.

Besonders geeignete Verbindungen c) sind solche, die die folgenden Strukturelemente enthalten: 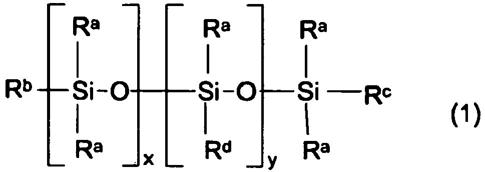 wobei:
die Reste R^{a} identisch oder unterschiedlich sein können, und ausgewählt sind unter Alkyl, Cycloalkyl, Cycloalkylalkyl, Aryl und Arylalkyl, insbesondere unter C₁-C₈-Alkyl, C₅-C₈-Cycloalkyl, Benzyl und Phenyl,
R^{b}, R^{c} und R^{d} unabhängig voneinander eine der zuvor für R^{a} angegebenen Bedeutungen haben oder für -(CH2)₁₋₆-OH, -(CH₂)₁₋₆-NHR^{e} oder einen Rest der Formel (1.1)

   -(CH₂)₁₋₆-O-(CH₂CH₂O)ₐ(CH₂CH(CH₃)O)_{b}-(C=O)_{c}-R^{f} (1.1)

   stehen, wobei
   in der Formel (1.1) die Reihenfolge der Alkylenoxideinheiten beliebig ist,
   a und b unabhängig voneinander für eine ganze Zahl von 0 bis 200 stehen, wobei die Summe aus a und b > 0 ist,
   c für 0 oder 1 steht,
R^{e} für Wasserstoff, C₁-C₈-Alkyl oder C₅-C₈-Cycloalkyl steht,
R^{f} für Wasserstoff, C₁-C₄₀-Alkyl, vorzugsweise Methyl, oder für den Fall, dass c = 0 ist für das Anion einer anorganischen Säure stehen kann,
mit der Maßgabe, dass wenigstens einer der Reste R^{b}, R^{c} und R^{d} für einen Rest der Formel (1.1) steht.

Vorzugsweise sind x und y so ausgewählt, dass das Molekulargewicht des Polysiloxan-Blocks zwischen 300 und 30000 liegt.

Bevorzugt steht der Rest R^{d} für einen Rest der Formel (1.1).

Bevorzugt stehen R^{b} und R^{c} für C₁-C₈-Alkyl, insbesondere für Methyl.

Bevorzugte Reste (1.1) sind solche, bei denen die Summe aus a+b zwischen 5 und 200 beträgt.

Bevorzugt werden die Gruppen R^{e} aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl, Xylyl und Resten der Formel (1.1).

Besonders geeignete Reste R^{f} sind solche, bei denen im Falle von c = 1 R^{f} ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R^{f} sind, für den Fall, dass c = 0 ist, Phosphat und Sulfat.

Besonders bevorzugte Siliconderivate c) sind solche, der allgemeinen Struktur: 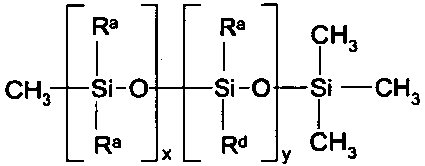 worin R^{d} für einen Rest der Formel (1.1)

-(CH2)₁₋₆-O-(CH₂CH₂O)ₐ(CH₂CH(CH₃)O)_{b}-(C=O)_{c}-R^{f} (1.1)

wie zuvor definiert steht. Speziell handelt es sich um Belsil ® DMC 6031 der Fa. Wacker.

In einer weiteren Ausführungsform handelt es sich bei der Siliconverbindung c) um eine α,β-ethylenisch ungesättigte Verbindung, die wenigstens eine Polysiloxangruppe aufweist.

Geeignete ethylenisch ungesättigte Monomere c) mit einer Polysiloxangruppe sind beispielsweise Verbindungen der allgemeinen Formel V: 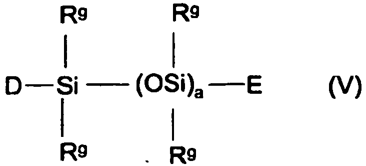 worin
- D: für eine ethylenisch ungesättigte Gruppe steht, die vorzugsweise ausgewählt ist unter Vinylgruppen, Vinyl(C₁-C₄-alkylen)gruppen, Acryloyloxy(C₁-C₄-alkylen)-gruppen und Methacryloyloxy(C₁-C₄-alkylen)gruppen,
- R^{g}: jeweils unabhängig voneinander für gleiche oder verschiedene Gruppen steht, die ausgewählt sind unter C₁-C₁₀-Alkyl, Phenyl, Benzyl, C₄-C₈-Cycloalkyl sowie Polyalkylen-, Polyoxyalkylen- und Polyalkylenimingruppen, die eine endständige Alkylether-, Ester- oder Amidfunktion aufweisen können,
- E: die für R⁹ oder die für D angegebenen Bedeutungen aufweisen kann und
- a: für eine ganze Zahl von 1 bis 1000, vorzugsweise 2 bis 250, steht.

Geeignete Verbindungen der Formel V sind z. B. in der EP-A-0 408 311 beschrieben, worauf hier in vollem Umfang Bezug genommen wird.

Weitere geeignete Siliconverbindungen c), die wenigstens eine radikalisch polymerisierbare Doppelbindung aufweisen, sind radikalisch polymerisierbare, Siloxangruppen-haltige Urethan(meth)acrylate. Geeignet sind beispielsweise die in der EP-A-0 274 699 beschriebenen (Meth)acrylat-funktionalisierten Organopolysiloxan-Urethan-Copolymere, die durch Umsetzung eines mit Aminogruppen funktionalisierten Polysiloxans mit Urethan(meth)acrylat-Oligomeren erhältlich sind. Auf die Offenbarung dieses Dokuments wird hier Bezug genommen.

Bevorzugt sind die in der WO 2004/055088 beschriebenen Verbindungen. Bevorzugt wird als Verbindung c) weiterhin wenigstens ein radikalisch polymerisierbares, Siloxangruppen-haltiges Urethan(meth)acrylat eingesetzt, wie es in der WO 00/12588 beschrieben wird. Dabei handelt es sich um Siloxangruppen-haltige Urethan(meth)-acrylate c), die
α) wenigstens eine Verbindung, die mindestens ein aktives Wasserstoffatom und mindestens eine radikalisch polymerisierbare, α,β-ethylenisch ungesättigte Doppelbindung pro Molekül enthält,
β) wenigstens ein Diisocyanat,
γ) wenigstens eine Verbindung, die zwei aktive Wasserstoffatome pro Molekül enthält,
δ) wenigstens eine Verbindung, die mindestens ein aktives Wasserstoffatom und mindestens eine Siloxangruppe pro Molekül enthält,
eingebaut enthalten, und die Salze davon.

Im Rahmen der vorliegenden Erfindung umfasst der Ausdruck "Urethan(meth)acrylate" ganz allgemein Verbindungen, die wenigstens eine olefinisch ungesättigte radikalisch polymerisierbare Doppelbindung aufweisen. Dazu zählen auch allylisch ungesättigte Verbindungen. Weiterhin umfasst der Ausdruck "Urethan(meth)acrylate" auch Verbindungen, die Harnstoffgruppen statt oder zusätzlich zu den Urethangruppen aufweisen. Harnstoffgruppen resultieren bei der Umsetzung einer Isocyanatgruppe mit einer primären oder sekundären Aminogruppe.

### Komponente α)

Geeignete Verbindungen α) sind z. B. die üblichen, dem Fachmann bekannten Vinylverbindungen, die zusätzlich wenigstens eine gegenüber Isocyanatgruppen reaktive Gruppe aufweisen, die vorzugsweise ausgewählt ist unter Hydroxylgruppen sowie primären und sekundären Aminogruppen. Dazu zählen z. B. die Ester α,β-ethylenisch ungesättigter Mono- und Dicarbonsäuren mit mindestens zweiwertigen Alkoholen. Als α,β-ethylenisch ungesättigte Mono- und/oder Dicarbonsäuren können z. B. Acrylsäure, Methacrylsäure, Fumarsäure, Maleinsäure, Crotonsäure, Itaconsäure etc. und Gemische davon eingesetzt werden. Geeignete Alkohole sind übliche Diole, Triole und Polyole, z. B. 1,2-Ethandiol, 1,3-Propandiol, 1,4-Butandiol, 1,5-Pentandiol, 1,6-Hexandiol, 1,10-Decandiol, Diethylenglykol, 2,2,4-Trimethylpentandiol-1,5, 2,2-Dimethylpropandiol-1,3, 1,4-Dimethylolcyclohexan, 1,6-Dimethylolcyclohexan, Glycerin, Trimethylolpropan, Erythrit, Pentaerythrit, Sorbit etc. Bei den Verbindungen a) handelt es sich dann z. B. um Hydroxymethyl(meth)acrylat, Hydroxyethylethacrylat, 2-Hydroxy-ethyl(meth)acrylat, 2-Hydroxypropyl(meth)acrylat, 3-Hydroxypropyl(meth)acrylat, 3-Hydroxybutyl(meth)acrylat, 4-Hydroxybutyl(meth)acrylat, 6-Hydroxyhexyl(meth)acrylat, 3-Hydroxy-2-ethylhexyl(meth)acrylat sowie um Di(meth)acrylsäureester des 1,1,1-Trimethylolpropans oder des Glycerins.

Geeignete Monomere α) sind weiterhin die Ester und Amide der zuvor genannten α,β-ethylenisch ungesättigten Mono- und Dicarbonsäuren mit C₂-C₁₂-Aminoalkoholen, die eine primäre oder sekundäre Aminogruppe aufweisen. Dazu zählen Aminoalkylacrylate und Aminoalkylmethacrylate und deren N-Monoalkylderivate, die z. B. einen N-C₁-C₈-Monoalkylrest tragen, wie Aminomethyl(meth)acrylat, Aminoethyl(meth)acrylat, N-Methylaminomethyl(meth)acrylat, N-Ethylaminomethyl(meth)acrylat, N-Ethylaminoethyl(meth)acrylat, N-(n-propyl)aminomethyl(meth)acrylat, N-Isopropylaminomethyl(meth)acrylat und bevorzugt tert.-Butylaminoethylacrylat und tert.-Butylaminoethylmethacrylat. Dazu zählen weiterhin N-(Hydroxy-C₁-C₁₂-alkyl)(meth)acrylamide, wie N-Hydroxymethyl(meth)acrylamid, N-Hydroxyethyl(meth)acrylamid etc.

Geeignete Monomere α) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure, wie Aminomethyl(meth)acrylamid, Aminoethyl(meth)acrylamid, Aminopropyl(meth)acrylamid, Amino-n-butyl(meth)acrylamid, Methylaminoethyl(meth)acrylamid, Ethylaminoethyl(meth)acrylamid, Methylaminopropyl(meth)acrylamid, Ethylaminopropyl(meth)acrylamid, Methylamino-n-butyl(meth)acrylamid etc.

Geeignete Monomere α) sind auch die Reaktionsprodukte von Epoxidverbindungen, die mindestens eine Epoxidgruppe aufweisen, mit den zuvor genannten α,β-ethylenisch ungesättigten Mono- und/oder Dicarbonsäuren und deren Anhydriden. Geeignete Epoxidverbindungen sind z. B. Glycidylether, wie Bisphenol-A-diglycidylether, Resorcindiglycidylether, 1,3-Propandioldiglycidylether, 1,4-Butandioldiglycidylether, 1,5-Pentandioldiglycidylether, 1,6-Hexandioldiglycidylether etc.

### Komponente β)

Bei der Komponente β) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon. Bevorzugt handelt es sich bei der Komponente β) um Hexamethylendiisocyanat, Isophorondiisocyanat, o- und m-Xylylendiisocyanat, Dicyclohexylmethandiisocyanat und Mischungen davon. Gewünschtenfalls können bis zu 3 Mol-% der genannten Verbindungen durch Triisocyanate ersetzt sein.

### Komponente γ)

Geeignete Verbindungen der Komponente γ) sind z. B. Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Geeignete Diole γ) sind z. B. Ethylenglykol, Propylenglykol, Butylenglykol, Neopentylglykol, Cydohexandimethylol, Di-, Tri-, Tetra-, Penta- oder Hexaethylenglykol und Mischungen davon. Bevorzugt werden Neopentylglykol und/oder Cydohexandimethylol eingesetzt.

Geeignete Aminoalkohole γ) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminapentan-2-ol etc.

Geeignete Diamine γ) sind z. B. Ethylendiamin, Propylendiamin, 1,4-Diaminobutan, 1,5-Diaminopentan und 1,6-Diaminohexan.

Bevorzugte Verbindungen der Komponente γ) sind Polymerisate mit einem zahlenmittleren Molekulargewicht im Bereich von etwa 300 bis 5000, bevorzugt etwa 400 bis 4000, insbesondere 500 bis 3000. Dazu zählen z. B. Polyesterdiole, Polyetherole, α,ω-Diaminopolyether und Mischungen davon. Vorzugsweise werden Ethergruppenhaltige Polymerisate eingesetzt.

Bei den Polyetherolen γ) handelt es sich vorzugsweise um Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten.

Geeignete α,ω-Diaminopolyether γ) sind z. B. durch Aminierung von Polyalkylenoxiden mit Ammoniak herstellbar.

Geeignete Polytetrahydrofurane γ) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Brauchbare Polyesterdiole γ) weisen vorzugsweise ein zahlenmittleres Molekulargewicht im Bereich von etwa 400 bis 5000, bevorzugt 500 bis 3000, insbesondere 600 bis 2000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel

HO-C(-R')(-COOR")-OH

worin R' für H oder CH₃ steht und R" für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/- 1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan.

Die Verbindungen der Komponente γ) können einzeln oder als Mischungen eingesetzt werden.

### Komponente δ)

Bevorzugt ist die Komponente δ) ausgewählt unter:
- Polysiloxanen der allgemeinen Formel VI. 1 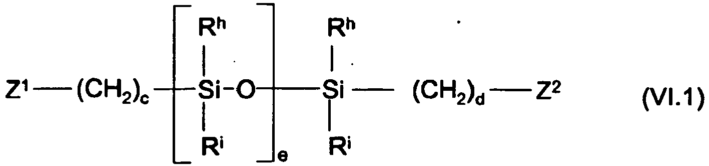 worin
   c und d unabhängig voneinander für 2 bis 8 stehen,
   e für 3 bis 100 steht,
   R^{h} und Rⁱ unabhängig voneinander für C₁-C₈-Alkyl, Benzyl oder Phenyl stehen, Z¹ und Z² unabhängig voneinander für OH, NHR^{k} oder einen Rest der Formel VII

   -O-(CH₂CH₂O)ᵥ(CH₂CH(CH₃)O)_{w}-H (VII)

   stehen, wobei
   in der Formel VII die Reihenfolge der Alkylenoxideinheiten beliebig ist und v und w unabhängig voneinander für eine ganze Zahl von 0 bis 200 stehen, wobei die Summe aus v und w > 0 ist,
   R^{k} für Wasserstoff, C₁-C₈-Alkyl oder C₅C₈-Cycloalkyl steht;
- Polysiloxanen der allgemeinen Formel VI.2 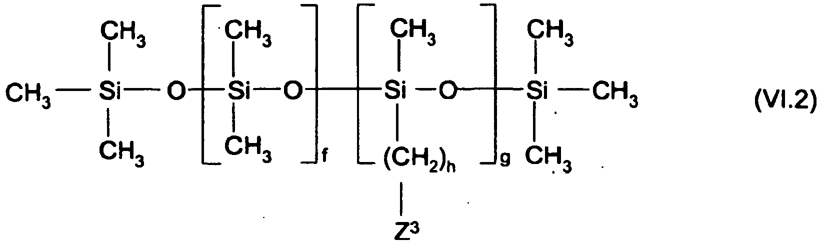 worin
   die Reihenfolge der Siloxaneinheiten beliebig ist,
   - f und g: unabhängig voneinander für 0 bis 100 stehen, wobei die Summe aus f und g mindestens 2 ist,
   - h: für eine ganze Zahl von 2 bis 8 steht,
   - Z³: für OH, NHR^{k} oder einen Rest der Formel VII steht,
   wobei R^{k} für Wasserstoff, C₁-C₈-Alkyl, C₅-C₈-Cycloalkyl oder einen Rest der Formel -(CH₂)ᵤ-NH₂ steht, wobei u für eine ganze Zahl von 1 bis 10, bevorzugt 2 bis 6, steht,
- Polysiloxanen mit sich wiederholenden Einheiten der allgemeinen Formel VI.3 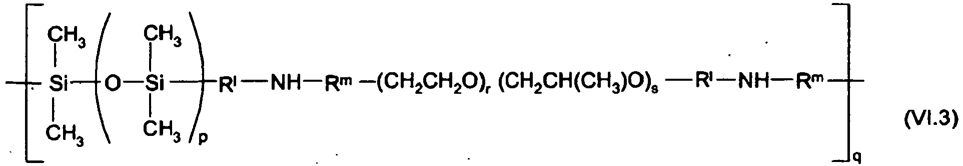 worin
   p für eine ganze Zahl von 0 bis 100 steht,
   q für eine ganze Zahl von 1 bis 8 steht,
   R^{l} und R^{m} unabhängig voneinander für C₁-C₈-Alkylen stehen,
   die Reihenfolge der Alkylenoxideinheiten beliebig ist und r und s unabhängig voneinander für eine ganze Zahl von 0 bis 200 stehen, wobei die Summe aus r und s > 0 ist,
- Polysiloxanen der allgemeinen Formel VI.4 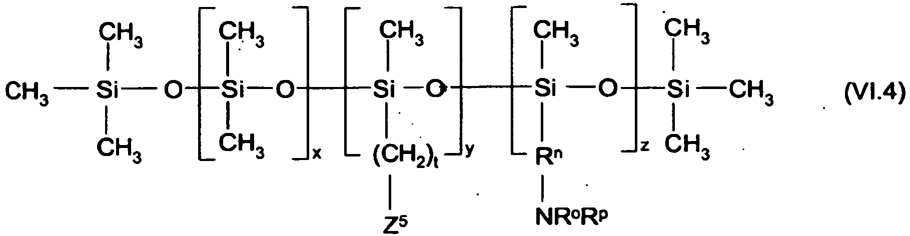 worin
   - Rⁿ: für einen C₁-C₈-Alkylenrest steht,
   - R^{o} und R^{p}: unabhängig voneinander für Wasserstoff, C₁-C₈-Alkyl oder C₅-C₈-Cycloalkyl stehen,
   die Reihenfolge der Siloxaneinheiten beliebig ist,
   x, y und z unabhängig voneinander für 0 bis 100 stehen, wobei die Summe aus x, y und z mindestens 3 ist,
   - t: für eine ganze Zahl von 2 bis 8 steht,
   - Z⁵: für einen Rest der Formel VIII

   -(OCH₂CH₂)ᵢ(OCH₂CH(CH₃))ⱼ-R^{q} (VIII)

   steht, worin
   die Reihenfolge der Alkylenoxideinheiten beliebig ist und i und j unabhängig voneinander für eine ganze Zahl von 0 bis 200 stehen, wobei die Summe aus i und j > O ist,
   - R^{q}: für Wasserstoff oder einen C₁-C₈-Alkylrest steht.
   und Mischungen davon.

Nach einer geeigneten Ausführungsform weisen die Polysiloxane δ) der allgemeinen Formel VI.1 keine Alkylenoxidreste der allgemeinen Formel VII auf. Diese Polysiloxane c4) weisen dann vorzugsweise ein zahlenmittleres Molekulargewicht im Bereich von etwa 300 bis 5000, bevorzugt 400 bis 3000 auf.

Geeignete Polysiloxane δ), die keine Alkylenoxidreste aufweisen sind z. B. die Tegomer®-Marken der Fa. Goldschmidt.

Nach einer weiteren geeigneten Ausführungsform handelt es sich bei den Polysiloxanen δ) um Silioonpoly(alkylenoxid)-Copolymere der Formel VI.1, wobei wenigstens einer oder beide Reste Z¹ und/oder Z² für einen Rest der allgemeinen Formel VII stehen.

Vorzugsweise ist in der Formel VII die Summe aus v und w so gewählt, dass das Molekulargewicht der Polysiloxane δ) dann in einem Bereich von etwa 300 bis 30000 liegt.

Bevorzugt liegt die Gesamtzahl der Alkylenoxideinheiten der Polysiloxane δ), d. h. die Summe aus v und w in der Formel VII dann in einem Bereich von etwa 3 bis 200, bevorzugt 5 bis 180.

Nach einer weiteren geeigneten Ausführungsform handelt es sich bei den Polysiloxanen δ) um Siliconpoly(alkylenoxid)-Copolymere der Formel VI.2, die wenigstens einen Rest Z³ der allgemeinen Formel VII aufweisen.

Vorzugsweise ist dann in der Formel VII die Summe aus v und w wiederum so gewählt, dass das Molekulargewicht der Polysiloxane δ) dann in einem Bereich von etwa 300 bis 30000 liegt. Bevorzugt liegt die Gesamtzahl der Alkylenoxideinheiten der Polysiloxane δ), d. h. die Summe aus v und w in der Formel VII dann ebenfalls in einem Bereich von etwa 3 bis 200, bevorzugt 5 bis 180.

Geeignete Siliconpoly(alkylenoxid)-Copolymere δ), die unter dem internationalen Freinamen Dimethicon bekannt sind, sind die Tegopren®-Marken der Fa. Goldschmidt, Belsil® 6031 der Fa. Wacker und Silvet® L der Fa. Witco.

Nach einer bevorzugten Ausführungsform handelt es sich bei den Polysiloxanen δ) um Siliconpoly(alkylenoxid)-Copolymere der Formel VI.2, die wenigstens einen Rest Z³ aufweisen, worin Z³ für NHR^{k} steht und R^{k} für Wasserstoff oder einen Rest der Formel -(CH₂)ᵤ-NH₂ steht. u steht für eine ganze Zahl von 1 bis 10, bevorzugt 2 bis 6. Dazu zählen z. B. die MAN- und MAR-Marken der Fa. Hüls sowie die Finish-Marken der Fa. Wacker, z. B. Finish WT 1270.

Bevorzugt umfassen die Polysiloxane δ) wenigstens eine Verbindung der allgemeinen Formel VI.3. Bevorzugt stehen in der Formel VI.3 R^{l} und R^{m} unabhängig voneinander für einen C₂-C₄-Alkylenrest. Insbesondere stehen R^{l} und R^{m} unabhängig voneinander für einen C₂-C₃-Alkylenrest.

Vorzugsweise liegt das Molekulargewicht der Verbindung der Formel VI.3 in einem Bereich von etwa 300 bis 100000 liegt.

Vorzugsweise steht in der Formel VI.3 p für eine ganze Zahl von 1 bis 20, wie z. B. 2 bis 10.

Bevorzugt liegt die Gesamtzahl der Alkylenoxideinheiten der Verbindung der Formel VI.3, d. h. die Summe aus r und s, in einem Bereich von etwa 3 bis 200, bevorzugt 5 bis 180.

Bevorzugt sind die Endgruppen der Polysiloxane mit Wiederholungseinheiten der allgemeinen Formel VI.3 ausgewählt unter (CH₃)₃SiO, H, C₁-C₈-Alkyl und Mischungen davon.

Aminogruppen-haltige Verbindungen mit Wiederholungseinheiten der allgemeinen Formel VI.3 weisen bevorzugt eine Aminzahl in einem Bereich von etwa 2 bis 50, insbesondere 3 bis 20 auf.

Geeignete alkoxylierte Siloxanamine der Formel VI.3 sind z. B. in derWO-A-97/32917 beschrieben, auf die hier in vollem Umfang Bezug genommen wird. Kommerziell erhältliche Verbindungen sind z. B. die Silsoft®-Marken der Fa. Witco, z. B. Silsoft® A-843.

Bevorzugt steht in der Formel VI.4 der Rest Rⁿ für einen C₂-C₄-Alkylenrest.

Bevorzugt stehen in der Formel VI.4 R^{o} und R^{p} unabhängig voneinander für Wasserstoff oder C₁-C₄-Alkyl.

Vorzugsweise wird die Summe aus x, y und z so gewählt, dass das Molekulargewicht der Verbindung der Formel VI.4 in einem Bereich von etwa 300 bis 100000, bevorzugt 500 bis 50000, liegt.

Bevorzugt liegt die Gesamtzahl der Alkylenoxideinheiten des Restes der Formel VIII, d. h. die Summe aus i und j, in einem Bereich von etwa 3 bis 200, bevorzugt 5 bis 80.

Bevorzugt steht in der Formel VIII der Rest R^{q} für Wasserstoff oder C₁-C₄-Alkyl.

Eine geeignete Verbindung der Formel VI.4 ist z. B. Silsoft® A-858 der Fa. Witco.

Geeignete Polysiloxane δ) sind auch die in der EP-A-277 816 beschriebenen Polydimethylsiloxane.

Gegebenenfalls enthalten die erfindungsgemäßen Urethan(meth)acrylate zusätzlich wenigstens eine Komponente eingebaut, die ausgewählt ist unter
ε) Verbindungen, die zwei oder mehrere aktive Wasserstoffatome und mindestens eine ionogene und/oder ionische Gruppe pro Molekül enthalten,
ζ) einwertigen Alkoholen, Aminen mit einer primären oder sekundären Aminogruppe, aliphatischen, cycloaliphatischen oder aromatischen Monoisocyanaten und Mischungen davon,
η) α,β-ethytenisch ungesättigten Verbindungen, die zusätzlich wenigstens eine Isocyanatgruppe pro Molekül enthalten,
und Mischungen davon.

### Monomer d)

In einer bevorzugten Ausführungsform enthalten die erfindungsgemäßen Copolymere zusätzlich zu den zuvor genannten Monomeren a) bis c) N-Vinylpyrrolidon und/oder N-Vinylcaprolactam einpolymerisiert.

Die erfindungsgemäß verwendeten Copolymere enthalten vorzugsweise 5 bis 95 Gew.%, besonders bevorzugt 10 bis 90 Gew.-%, bezogen auf das Gesamtgewicht der zur Polymerisation eingesetzten Verbindungen, wenigstens eines Monomers d) einpolymerisiert.

Eine spezielle Ausführung betrifft die Verwendung von Copolymeren, die kein Monomer d) einpolymerisiert enthalten. Dazu zählen insbesondere Copolymere, die kein N-Vinylpyrrolidon und/oder kein N-Vinylcaprolactam einpolymerisiert enthalten.

### Monomer e)

Die erfindungsgemäßen Copolymere A) können zusätzlich wenigstens ein hydrophobes Monomer e) einpolymerisiert enthalten.

Vorzugsweise enthalten Silicongruppen-freie Copolymere 0,2 bis 40 Gew.-%, besonders bevorzugt 0,5 bis 40 Gew.-%, insbesondere 1 bis 30 Gew.-%, bezogen auf das Gesamtgewicht der zur Polymerisation eingesetzten Verbindungen, wenigstens eines hydrophoben Monomers e) (und/oder wenigstens eines hydrophoben Monomers f) einpolymerisiert). Vorzugsweise enthalten Silicongruppen-haltige Copolymere 0,1 bis 30 Gew.-%, besonders bevorzugt 0,2 bis 20 Gew.-%, insbesondere 0,5 bis 15 Gew.-%, bezogen auf das Gesamtgewicht der zur Polymerisation eingesetzten Verbindungen, wenigstens eines hydrophoben Monomers e) einpolymerisiert.

Geeignete Monomere e) sind z. B. n-Octyl(meth)acrylat,1,1,3,3-Tetramethylbutyl(meth)acylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon.

GeeigneteMonomere e) sind C₈-C₂₂-Alkylvinylether, z. B. n-Octylvinylether, 1,1,3,3-Tetramethylbutylvinylether, Ethylhexylvinylether, n-Nonylvinylether, n-Decylvinylether, n-Undecylvinylether, Tridecylvinylether, Myristylvinylether, Pentadecylvinylether, Palmitylvinylether, Heptadecylvinylether, Octadecylvinylether, Nonadecylvinylether, Arrachinylvinylether, Behenylvinylether, Lignocerenylvinylether, Cerotinylvinylether, Melissinylvinylether, Palmitoleinylvinylether, Oleylvinylether, Linolylvinylether, Linolenylvinylether, Stearylvinylether, Laurylvinylether und MIschungen davon.

Geeignete Polyetheracrylate e) sind z. B. die Polykondensationsprodukte der zuvor genannten α,β-ethylenisch ungesättigten Mono- und/oder Dicarbonsäuren und deren Säurechloriden, -amiden und -anhydriden mit Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Polyetheracrylate e) können allein oder in Mischungen zur Herstellung der erfindungsgemäß eingesetzten Polymere verwendet werden.

Geeignete Allylalkoholalkoxilate e) sind z. B. die Veretherungsprodukte von Allylchlorid mit entsprechenden Polyetherolen. Geeignete Polyetherole können leicht durch Umsetzung von Ethylenoxid, 1,2-Propylenoxid und/oder Epichlorhydrin mit einem Starteralkohol R⁹-OH hergestellt werden. Die Alkylenoxide können einzeln, alternierend nacheinander oder als Mischung eingesetzt werden. Die Allylalkoholalkoxilate e) können allein oder in Mischungen zur Herstellung der erfindungsgemäß eingesetzten Polymere verwendet werden.

Geeignete Monomere e) sind C₈-C₂₂-Carbonsäurevinylester. Dazu zählen z. B. n-Octylvinylester, 1,1,3,3-Tetramethylbutylvinylester, Ethylhexylvinylester, n-Nonylvinylester, n-Decylvinylester, n-Undecylvinylester, Tridecylvinylester, Myristylvinylester, Pentadecylvinylester, Palmitylvinylester, Heptadecylvinylester, Octadecylvinylester, Nonadecylvinylester, Arrachinylvinylester, Behenylvinylester, Lignocerenylvinylester, Cerotinylvinylester, Melissinylvinylester, Palmitoleinylvinylester, Oleylvinylester, Linolylvinylester, Linolenylvinylester, Stearylvinylester, Laurylvinylester und Mischungen davon.

### Monomer f)

Die erfindungsgemäß verwendeten Copolymere können zusätzlich wenigstens ein Monomer f) einpolymerisiert enthalten, dass ausgewählt ist unter Methyl(meth)acrylat, Ethyl(meth)acrylat, n-Butyl(meth)acrylat und Mischungen davon. Der Anteil an Monomeren f) beträgt bis zu 40 Gew.-%, bezogen auf das Gesamtgewicht der zur Polymerisation eingesetzten Verbindungen. Eine geeignete Einsatzmenge für zusätzliche Monomere f) liegt in einem Bereich von 0,1 bis 30 Gew.%, insbesondere 1 bis 25 Gew.-%, bezogen auf das Gesamtgewicht der zur Polymerisation eingesetzten Verbindungen.

Eine spezielle Ausführungsform ist die Verwendung ampholytischer Copolymere, die kein amidgruppenhaltiges Monomer d) einpolymerisiert enthalten. Dazu zählen speziell Copolymere, die kein Vinylpyrrolidon und/oder kein Vinylcaprolactam einpolymerisiert enthalten. Eine ganz spezielle Ausführung ist die erfindungsgemäße Verwendung vinylpyrrolidonfreier Copolymere.

Bevorzugt ist die Verwendung eines Copolymers, das kein Monomer d) (speziell kein Vinylpyrrolidon und/oder Vinylcaprolactam) einpolymerisiert enthält, das
- 2 bis 96 Gew-% Acrylsäure und/oder Methacrylsäure,
- 1 bis 30 Gew.-% wenigstens einer N-Vinylimidazol-Verbindung,
- 0,1 bis 2 Gew.-% Ethylenglykoldi(meth)acrylat und/oder Pentaerythrittriallylether,
- 0 bis 15 Gew.-%, wenigstens einer Siliconverbindung c),
einpolymerisiert enthält.

Die zuvor genannten Copolymere können zusätzlich
- bis zu 40 Gew.-% wenigstens einer Verbindung e) die ausgewählt ist unter C₈-C₂₂-(meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon, und/oder
- bis zu 40 Gew.-% wenigstens eines Monomers f), das ausgewählt ist unter Methyl(meth)acrylat, Ethyl(meth)acrylat, n-Butyl(meth)acrylat und Mischungen davon,
einpolymerisiert enthalten.

Eine spezielle Ausführungsform ist die Verwendung anionisch ampholytischer Copolymere.

Bevorzugt sind weiterhin anionisch ampholytische Copolymere A), zu deren Herstellung zumindest ein Teil der Monomere a1) und a2) in Form eines Monomerenpaares eingesetzt wird.

Eine spezielle Ausführungsform ist die Verwendung Silicongruppen-freier anionisch ampholytischer Copolymere.

Besonders bevorzugt ist die Verwendung eines ampholytischen Copolymers, das erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.% Methacrylsäure und/oder Acrylsäure,
- 0,1 bis 2 Gew.-% Ethylenglykoldi(meth)acrylat und/oder Pentaerythrittriallylether,
- 20 bis 95 Gew.-% Vinylpyrrolidon und/oder Vinylcaprolactam,
- 0 bis 40 Gew.-% wenigstens eines weiteren Monomers, das ausgewählt ist unter Methyl(meth)acrylat, Ethyl(meth)acrylat, n-Butyl(meth)acrylat, C₈-C₂₂-(Meth)acrylaten, mit C₈-C₂₂Alkylgruppen terminierten Polyether(meth)acrylaten, C₈-C₂₂-Carbonsäurevinylestern und Mischungen davon.

Besonders bevorzugt ist weiterhin die Verwendung eines ampholytischen Copolymers, das erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinyllmidazol und Acrylsäure und/oder Methacrylsäure,
- 5 bis 70 Gew.-% Methacrylsäure und/oder Acrylsäure,
- 0,1 bis 2 Gew.-% Ethylenglykoldi(meth)acrylat und/oder Pentaerythrittriallylether,
- 20 bis 85 Gew.-% Vinylpyrrolidon und/oder Vinylcaprolactam.

Besonders bevorzugt Ist weiterhin die Verwendung eines ampholytischen Copolymers, das erhältlich ist durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.-% Methacrylsäure und/oder Acrylsäure,
- 0,1 bis 2 Gew.-% Ethylenglykoldi(meth)acrylat und/oder Pentaerythrittriallylether,
- 20 bis 85 Gew.-% Vinylpyrrolidon und/oder Vinylcaprolactam,
- 5 bis 40 Gew.-% wenigstens eines weiteren Monomers, das ausgewählt ist unter C₁-C₈-(Meth)acrylaten, insbesondere Methyl(meth)acrylat, Ethyl(meth)acrylat, n-Butyl(meth)acrylat und Mischungen davon.

Besonders bevorzugt ist weiterhin die Verwendung eines ampholytischen Copolymers, das erhältlich ist durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.-% Methacrylsäure und/oder Acrylsäure,
- 0,1 bis 2 Gew.-% Ethylenglykoldi(meth)acrylat und/oder Pentaerythrittriallylether,
- 20 bis 85 Gew.-% Vinylpyrrolidon und/oder Vinylcaprolactam,
- 0,5 bis 20 Gew.-%, vorzugsweise 1 bis 10 Gew.%, wenigstens eines weiteren Monomers, das ausgewählt ist unter C₈-C₃₀-(Meth)acrylaten, mit C₈-C₃₀-Alkylgruppen terminierten Polyether(meth)acrylaten und Mischungen davon, insbesondere unter Stearylmethacrylat, mit C₁₈-C₂₂Alkylgruppen terminierten Polyethylenglycol(meth)acrylaten und Mischungen davon.

In einer speziellen Ausführung werden alle zuvor genannten Silicongruppen-freien, anionischen, ampholytischen Copolymere, die wenigstens 5 Gew.-% mindestens einer Vinylimidazol-Verbindung einpolymerisiert enthalten, einer teilweisen oder vollständigen Quaternisierung unterzogen. Geeignete Quaternisierungsmittel sind die im Folgenden genannten.

Besonders bevorzugt ist die Verwendung eines Silicongruppen-haltigen ampholytischen Copolymers, das erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 5 bis 70 Gew.-% Methacrylsäure und/oder Acrylsäure a1),
- 0,1 bis 2 Gew.-% Ethylenglykoldi(meth)acrylat und/oder Pentaerythrittriallylether,
- 0,1 bis 15 Gew.-%, wenigstens einer Siliconverbindung c),
- 20 bis 95 Gew.-% Vinylpyrrolidon und/oder Vinylcaprolactam d),
- 0,1 bis 20 Gew.-% wenigstens einer Verbindung e), die ausgewählt ist unter C₈-C₂₂-(Meth)acrylaten, C₈-C₂₂-Alkylvinylether, mit C₈-C₂₂-Alkylgruppen terminierten Polyether(meth)acrylaten, mit C₈-C₂₂-Alkylgruppen terminierten Allylalkoholalkoxilaten, C₈-C₂₂"Carbonsäurevinylestern und Mischungen davon,
- 5 bis 40 Gew.-% wenigstens eines Monomers f), das ausgewählt ist unter Methyl(meth)acrylat, Ethyl(meth)acrylat, n-Butyl(meth)acrylat und Mischungen davon.

Besonders bevorzugt ist weiterhin die Verwendung eines Silicongruppen-haltigen ampholytischen Copolymers, das erhältlich ist durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 5 bis 70 Gew.-% Methacrylsäure und/oder Acrylsäure a1).
- 0,1 bis 2 Gew.-% Ethylenglykoldi(meth)acrylat und/oder Pentaerythrittriallylether,
- 0,1 bis 15 Gew.-%, wenigstens einer Siliconverbindung c),
- 20 bis 85 Gew.-% Vinylpyrrolidon und/oder Vinylcaprolactam d).

Besonders bevorzugt ist weiterhin die Verwendung eines Silicongruppen-haltigen ampholytischen Copolymers, das erhältlich ist durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1).
- 5 bis 70 Gew.-% Methacrylsäure und/oder Acrylsäure a1).
- 0,1 bis 2 Gew.-% Ethylenglykoldi(meth)acrylat und/oder Pentaerythrittriallylether.
- 0,1 bis 15 Gew.-%, wenigstens einer Siliconverbindung c),
- 20 bis 85 Gew.-% Vinylpyrrolidon und/oder Vinylcaprolactam d),
- 5 bis 40 Gew.-% wenigstens eines weiteren Monomers f), das ausgewählt ist unter Methyl(meth)acrylat, Ethyl(meth)acrylat, n-Butyl(meth)acrylat und Mischungen davon.

Besonders bevorzugt ist weiterhin die Verwendung eines Silicongruppen-haltigen ampholytischen Copolymers, das erhältlich ist durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 5 bis 70 Gew.-% Methacrylsäure und/oder Acrylsäure a1),
- 0,1 bis 2 Gew.-% Ethylenglykoldi(meth)acrylat und/oder Pentaerythrittriallylether,
- 0,1 bis 15 Gew.-%, wenigstens einer Siliconverbindung c),
- 20 bis 85 Gew.-% Vinylpyrrolidon und/oder Vinylcaprolactam d),
- 0,5 bis 20 Gew.-%, vorzugsweise 1 bis 10 Gew.-%, wenigstens eines weiteren Monomers, das ausgewählt ist unter C₈-C₂₂-(Meth)acrylaten, C₈-C₂₂-Alkylvinylether, mit C₈-C₂₂-Alkylgruppen terminierten Polyether(meth)acrylaten, mit C₈-C₂₂-Alkylgruppen terminierten Allylalkoholalkoxilaten und Mischungen davon, insbesondere unter Stearylmethacrylat, mit C₈-C₂₂-Alkylgruppen terminierten Polyethylenglycol(meth)acrylaten und Mischungen davon.

In einer speziellen Ausführung werden alle zuvor genannten anionischen Silicongruppen-haltigen Copolymere A), die wenigstens 5 Gew.-% mindestens einer Vinylimidazol-Verbindung einpolymerisiert enthalten, einer teilweisen oder vollständigen Quatemisierung unterzogen. Geeignete Quaternisierungsmittel sind die im Folgenden genannten.

Eine weitere spezielle Ausführungsform ist die Verwendung kationisch ampholytischer Copolymere.

Bevorzugte kationisch ampholytische Copolymere A) sind erhältlich durch radikalische Copolymerisation von
- Methacrylsäure und/oder Acrylsäure a1),
- N-Vinylimidazol a2),
- Ethylenglykoldi(meth)acrylat und/oder Pentaerythrittriallylether,
- gegebenenfalls wenigstens einer Siliconverbindung c),
wobei das molare Verhältnis von kationogenen/kationischen Gruppen zu anionogenen/anionischen Gruppen wenigstens 6 : 1 beträgt.

Bevorzugte kationisch ampholytische Copolymere A) sind erhältlich durch radikalische Copolymerisation von
- Methacrylsäure und/oder Acrylsäure a1),
- N-Vinylimidazol a2),
- Ethylenglykoldi(meth)acylat
- und/oder Pentaerythrittriallylether,
- gegebenenfalls wenigstens einer Siliconverbindung c),
- 3 bis 35 Gew.-%, bezogen auf das Gesamtgewicht der zur Polymerisation eingesetzten Monomere, wenigstens eines Monomers f),
wobei das molare Verhältnis von kationogenen/kationischen Gruppen zu anionogenen/anionischen Gruppen wenigstens 6 : 1 beträgt.

Bevorzugte kationisch ampholytische Copolymere A) sind erhältlich durch radikalische Copolymerisation von
- Methacrylsäure und/oder Acrylsäure a1),
- N-Vinylimidazol a2),
- Ethylenglykoldi(meth)acrylat und/oder Pentaerythrittriallylether,
- gegebenenfalls wenigstens einer Siliconverbindung c),
- 0,1 bis 10 Gew.-% wenigstens einer Verbindung e), die ausgewählt ist unter C₈-C₂₂-(Meth)acrylaten, C₈-C₂₂-Alkylvinylethern, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
wobei das molare Verhältnis von kationogenen/kationischen Gruppen zu anionogenen/anionischen Gruppen wenigstens 6 : 1 beträgt.

Bevorzugt sind weiterhin kationisch ampholytische Copolymere A), zu deren Herstellung zumindest ein Teil der Monomere a1) und a2) in Form eines Monomerenpaares eingesetzt werden.

Eine spezielle Ausführungsform ist die Verwendung Silicongruppen-freier kationisch ampholytischer Copolymere.

Besonders bevorzugt ist die Verwendung eines ampholytischen Copolymers, das erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% wenigstens einer Vinylimidazol-Verbindung
- 0,1 bis 2 Gew.-% Ethylenglykoldi(meth)acrylat und/oder Pentaerythrittriallylether,
- 0 bis 95 Gew.-%, vorzugsweise 20 bis 95 Gew.-% Vinylpyrrolidon und/oder Vinylcaprolactam,
- 0 bis 40 Gew.-% wenigstens eines weiteren Monomers, das ausgewählt ist unter Methyl(meth)acrylat, Ethyl(meth)acrylat, n-Butyl(meth)acrylat, C₈-C₂₂-(Meth)acrylaten, mit C₈-C₂₂-Alkylgruppen terminierten Polyether(meth)acylaten, C₈-C₂₂-Carbonsäurevinylestern und Mischungen davon.

Besonders bevorzugt ist weiterhin die Verwendung eines ampholytischen Copolymers, das erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% wenigstens einer Vinylimidazol-Verbindung
- 0,1 bis 2 Gew.-% Ethylenglykoldi(meth)acrylat und/oder Pentaerythrittriallylether,
- 20 bis 85 Gew.-% Vinylpyrrolidon und/oder Vinylcaprolactam.

Besonders bevorzugt ist die Verwendung eines ampholytischen Copolymers, das erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 50 Gew.-% wenigstens einer Vinylimidazol-Verbindung
- 0,1 bis 2 Gew.-% Ethylenglykoldi(meth)acrylat und/oder Pentaerythrittriallylether,
- 20 bis 95 Gew.-% Vinylpyrrolidon und/oder Vinylcaprolactam,
- 5 bis 40 Gew.-% wenigstens eines weiteren Monomers, das ausgewählt ist unter C₁-C₈-(Meth)acrylaten, insbesondere Methyl(meth)acrylat, Ethyl(meth)acrylat, n-Butyl(meth)acrylat und Mischungen davon.

Besonders bevorzugt ist die Verwendung eines ampholytischen Copolymers, das erhältlich ist durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 3 bis 70 Gew.-% wenigstens einer Vinylimidazol-Verbindung,
- 0,1 bis 2 Gew.-% Ethylenglykoldi(meth)acrylat und/oder Pentaerythrittriallylether,
- 20 bis 85 Gew.-% Vinylpyrrolidon und/oder Vinylcaprolactam,
- 0,5 bis 20 Gew.-%, vorzugsweise 1 bis 10 Gew.-%, wenigstens eines weiteren Monomers, das ausgewählt ist unter C₈-C₃₀-(Meth)acrylaten, mit C₈-C₃₀-Alkylgruppen terminierten Polyether(meth)acrylaten und Mischungen davon, insbesondere unter Stearylmethacrylat, mit C₁₈-C₂₂-Alkylgruppen terminierten Polyethylenglycol(meth)acrylaten und Mischungen davon.

In einer speziellen Ausführung werden alle zuvor genannten Silicongruppen-freien, kationischen, ampholytischen Copolymere einer teilweisen oder vollständigen Quadernisierung unterzogen. Geeignete Quaternisierungsmittel sind die im Folgenden genannten.

Besonders bevorzugt ist die Verwendung eines Silicongruppen-haltigen ampholytischen Copolymers, das erhältlich durch radikalische Copolymerisation von
- wenigstens 2 Gew.%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 3 bis 70 Gew.-% wenigstens einer Vinylimidazol-Verbindung,
- 0,1 bis 2 Gew.-% Ethylenglykoldi(meth)acrylat und/oder Pentaerythrittriallylether,
- 0,1 bis 15 Gew.-%, wenigstens einer Siliconverbindung c),
- 0 bis 95 Gew.-%, vorzugsweise 20 bis 95 Gew.-% Vinylpyrrolidon und/oder Vinylcaprolactam d),
- 0 bis 20 Gew.-% wenigstens einer Verbindung e), die ausgewählt ist unter C₈-C₂₂-(Meth)acrylaten, C₈-C₂₂-Alkylvinylether, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon,
- 0 bis 40 Gew.-% wenigstens eines Monomers f), das ausgewählt ist unter Methyl(meth)acrylat, Ethyl(meth)acrylat, n-Butyl(meth)acrylat und Mischungen davon.

Besonders bevorzugt ist weiterhin die Verwendung eines Silicongruppen-haltigen ampholytischen Copolymers, das erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 3 bis 70 Gew.-% wenigstens einer Vinylimidazol-verbindung,
- 0,1 bis 2 Gew.-% Ethylenglykoldi(meth)acrylat und/oder Pentaerythrittriallytether,
- 0,05 bis 30 Gew.-%, besonders bevorzugt 0,1 bis 20 Gew.-%, insbesondere 0,1 bis 15 Gew.-%, wenigstens einer Siliconverbindung c),
- 20 bis 95 Gew.-% Vinylpyrrolidon und/oder Vinylcaprolactam d).

Besonders bevorzugt ist weiterhin die Verwendung eines Silicongruppen-haltigen ampholytischen Copolymers, das erhältlich durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 3 bis 50 Gew.-% wenigstens einer VinylimidazolVerbindung,
- 0,1 bis 2 Gew.-% Ethylenglykoldi(meth)acrylat und/oder Pentaerythrittriallylether,
- 0,1 bis 15 Gew.-%, wenigstens einer Siliconverbindung c),
- 20 bis 95 Gew.-% Vinylpyrrolidon und/oder Vinylcaprolactam d),
- 5 bis 40 Gew.-% wenigstens eines Monomers f), das ausgewählt ist unter Methyl(meth)acrylat, Ethyl(meth)acrylat, n-Butylacrylat und Mischungen davon.

Besonders bevorzugt ist weiterhin die Verwendung eines Silicongruppen-haltigen ampholytischen Copolymers, das erhältlich durch radikalische Copolymerisation von
- wenigstens 5 Gew.-%, bezogen auf das Gesamtgewicht der zur Polymerisation eingesetzten Monomere, wenigstens eines Monomerenpaars aus N-Vinylimidazol a2) und Acrylsäure und/oder Methacrylsäure a1),
- 3 bis 70 Gew.-% wenigstens einer Vinylimidazol-Verbindung,
- 0,1 bis 2 Gew.-% Ethylenglykoldi(meth)acrylat und/oder Pentaerythrittriallylether,
- 0,1 bis 15 Gew.-%, wenigstens einer Siliconverbindung c),
- 20 bis 85 Gew.-% Vinylpyrrolidon und/oder Vinylcaprolactam,
- 0,5 bis 20 Gew.-%, vorzugsweise 1 bis 10 Gew.-%, wenigstens eines weiteren Monomers e), das ausgewählt ist unter C₈-C₂₂-(Meth)acrylaten, C₈-C₂₂-Alkylvinylether, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestern und Mischungen davon.

In einer speziellen Ausführung werden alle zuvor genannten kationischen Silicongruppen-haltigen Copolymere einer teilweisen oder vollständigen Quatemisierung unterzogen. Geeignete Quaternisierungsmittel sind die im Folgenden genannten. Ein besonders bevorzugtes Quaternisierungsmittel ist CH₃-Cl.

In einer speziellen Ausführung erfolgt die radikalische Copolymerisation der zuvor genannten Komponenten a1), a2), b) und, falls vorhanden, d) bis f) in Gegenwart wenigstens einer polyetherhaltigen Verbindung, die keine copolymerisierbare Doppelbindung aufweist. Dabei werden spezielle Copolymere mit vorteilhaften Eigenschaften erhalten. Dies kann beispielsweise auf eine Wirkung der Polyether-Komponente als Schutzkolloid oder Emulgator zurückzuführen sein. Dies kann beispielsweise auch aus einer zumindest teilweisen Pfropfung auf die Polyether-Komponente als Pfropfgrundlage resultieren. Es sind jedoch auch andere Mechanismen als eine Pfropfung vorstellbar. Die erfindungsgemäß verwendeten Copolymere umfassen ganz allgemein die Verfahrensprodukte der radikalischen Copolymerisation, worunter z. B. reine Pfropfpolymerisate, Mischungen von Pfropfpolymerisaten mit ungepfropften Verbindungen der Polyether-Komponente sowie beliebige Mischungen verstanden werden.

Vorzugsweise beträgt die Einsatzmenge der Polyether-Komponente (falls vorhanden) 0.1 bis 50 Gew.-%, besonders bevorzugt 1 bis 25 Gew.-%, bezogen auf das Gesamtgewicht der zur Polymerisation eingesetzten Komponenten.

Geeignete Polyether-haltige Verbindungen sind beispielsweise wasserlösliche oder wasserdispergierbare nichtionische Polymere, die Alkylenoxid-Wiederholungseinheiten aufweisen. Vorzugsweise beträgt der Anteil an Alkylenoxid-Wiederholungseinheiten mindestens 30 Gew.-%, bezogen auf das Gesamtgewicht der Verbindung. Geeignete polyetherhaltige Verbindungen sind beispielsweise Polyalkylenglykole, wie sie üblicherweise auch als nichtionische Tenside eingesetzt werden. Geeignete Polyalkylenglykole weisen im Allgemeinen ein zahlenmittleres Molekulargewicht im Bereich von etwa 150 bis 100000, bevorzugt 300 bis 50000, besonders bevorzugt 500 bis 40000 auf. Geeignete Polyalkylenglykole sind beispielsweise Polyethylenglykole, Polypropylenglykole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form Blöcken einpolymerisiert enthalten. Vorteilhafterweise verwendet man Homopolymerisate des Ethylenoxids oder Copolymerisate, die Ethylenoxid enthalten. Vorzugsweise beträgt der Anteil an von Ethylenoxid abgeleiteten Wiederholungseinheiten 40 bis 99 Gew.-%. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid.

Die Herstellung der erfindungsgemäß verwendeten Copolymere erfolgt durch Fällungs polymerisation.

Die Fällungspolymerisation erfolgt vorzugsweise in einem weitgehend wasserfreien, aprotischen Lösungsmittel oder Lösungsmittelgemisch, vorzugsweise in Ethylacetat und/oder n-Butylacetat. Unter einem weitgehend wasserfreien, aprotischen Lösungsmittel oder Lösungsmittelgemisch wird ein Lösungsmittel oder Lösungsmittelgemisch mit einem Wassergehalt von höchstens 5 Gew.-% verstanden.

Bevorzugt erfolgt die Fällungspolymerisation bei einer Temperatur im Bereich von 70 bis 140 °C, bevorzugt 75 bis 100 °C, insbesondere von 80 bis 95 °C. Die resultierenden Polymerteilchen fallen aus der Reaktionslösung aus und können durch übliche Verfahren, wie Filtration mittels Unterdruck, isoliert werden. Zur Fällungspolymerisation können oberflächenaktive, polymere Verbindungen, vorzugsweise auf Polysiloxanbasis, eingesetzt werden. Bei der Fällungspolymerisation werden in der Regel Polymere mit höheren Molekulargewichten als bei der Lösungspolymerisation erhalten.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisutfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat. Bis-(o-toluoyl)peroxid. Didecanoylperoxid, Dioctanoylperoxid, Dilauroylperoxid, tert.-Butylperisobutyrat, tert.-Butylperacetat, Di-tert.-amylperoxid, tert.-Butylhydroperoxid, Azo-bis-isobutyronitril, Azo-bis-(2-amidinopropan)dihydrochlorid oder 2,2'-Azo-bis-(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu¹.

In einer speziellen Ausführung werden zur Herstellung der erfindungsgemäßen Copolymere wenigstens zwei Radikalinitiatoren eingesetzt, die eine im Wesentlichen unabhängige Initiierung in wenigstens zwei Phasen ermöglichen. Dabei können Copolymere mit besonders geringen Restmonomergehalten erzielt werden.

Bevorzugt werden zur Copolymerisation wenigstens zwei Initiatoren eingesetzt, deren Zerfallstemperaturen um wenigstens 10 °C voneinander verschieden sind. Im Rahmen der Erfindung ist die Zerfallstemperatur ist definiert als die Temperatur, bei der 50 % der Moleküle innerhalb von 2,5 Stunden in freie Radikale zerfallen. Vorzugsweise erfolgt die Copolymerisation bei dieser Vorgehensweise bis zum Abschluss der Fällung des Copolymers bei einer Temperatur größer oder gleich der niedrigeren Zerfallstemperatur und kleiner der höheren Zerfallstemperatur, und nach der Fällung erfolgt eine weitere Umsetzung bei einer Temperatur größer oder gleich der höheren Zerfallstemperatur.

Bevorzugt umfasst das erfindungsgemäße Verfahren eine erste Polymerisationsphase bei einer ersten Polymerisationstemperatur und eine zweite Polymerisationsphase bei einer zweiten Polymerisationstemperatur oberhalb der ersten Polymerisationstemperatur, wobei zur Polymerisation wenigstens zwei Initiatoren eingesetzt werden, deren Halbwertszeiten bei der ersten Polymerisationstemperatur sich so unterscheiden, dass wenigstens einer dieser Initiatoren während der ersten Polymerisationsphase in Radikale zerfällt und wenigstens einer dieser Initiatoren während der ersten Polymerisationsphase im Wesentlichen nicht in Radikale zerfällt und während der zweiten Polymerisationsphase in Radikale zerfällt. Vorzugsweise beginnt bei dieser Vorgehensweise die zweite Polymerisationsphase im Wesentlichen nach Fällung des Copolymers. Unter "im Wesentlichen" nach Fällung des Copolymers wird verstanden, dass das Copolymer vorzugsweise zu mindestens 80 Gew.-%, vorzugsweise mindestens 90 Gew.-%, insbesondere wenigstens 95 Gew.-%, bezogen auf das Gesamtgewicht des Copolymers, in gefällter Form vorliegt.

Die Halbwertszeit eines Initiators kann nach üblichen, dem Fachmann bekannten Verfahren bestimmt werden, wie z. B. in der Druckschrift "Initiators for high polymers", Akzo Nobel, Nr. 10737, beschrieben. Vorzugsweise liegt die Halbwertszeit des ersten Polymerisationsinitiators bei der ersten Polymerisationstemperatur und des zweiten Polymerisationsinitiators bei der zweiten Polymerisationstemperatur in einem Bereich von etwa 1 Minute bis 3 Stunden, besonders bevorzugt 5 Minuten bis 2,5 Stunden. Gewünschtenfalls können auch kürzere Halbwertszeiten z. B. von 1 Sekunde bis 1 Minute oder längere Halbwertszeiten als 3 Stunden zum Einsatz kommen, solange sichergestellt ist, dass der/die bei der höheren Temperatur zerfallende(n) Initiator(en) im Wesentlichen während der zweiten Polymerisationsphase in Radikale zerfällt.

Zusätzlich zu der ersten und zweiten Polymerisationsphase können weitere Polymerisationsphasen bei davon verschiedenen Polymerisationstemperaturen angewandt werden. So ist es z. B. möglich, eine erste Polymerisationsphase bei einer ersten Polymerisationstemperatur durchzuführen, die so gewählt ist, dass eine kontrollierte Polymerisation (d. h. z. B. unter Vermeidung eines unerwünschten Temperaturanstiegs durch die Reaktionswärme, einer zu hohen Reaktionsgeschwindigkeit, etc.) erfolgt. Anschließend kann sich z. B. eine Nachpolymerisation bei einer Temperatur anschließen, die oberhalb der ersten und unterhalb der zweiten Polymerisationstemperatur liegt und die so gewählt ist, dass der/die bei der höheren Temperatur zerfallende(n) Initiator(en) im Wesentlichen nicht in Radikale zerfallen. Nach Abschluss dieser Nachpolymerisation, zu der gewünschtenfalls nochmals der bei der niedrigeren Temperatur zerfallende Initiator und/oder ein anderer unter den Bedingungen der Nachpolymerisation zerfallender Initiator zugegeben werden kann, kann sich dann die zweite Polymerisationsphase anschließen.

Vorzugsweise enthält das eingesetzte Initiatorsystem wenigstens zwei Initiatoren, deren Zerfallstemperaturen sich um wenigstens 15 °C voneinander unterscheiden.

Der bei der niedrigeren Temperatur zerfallende Initiator weist vorzugsweise eine Zerfallstemperatur von 50 bis 100 °C auf.

Der bei der höheren Temperatur zerfallende Initiator weist vorzugsweise eine Zerfallstemperatur von 80 bis 150 °C auf.

Vorzugsweise wird der bei der höheren Temperatur zerfallende Initiator zu Beginn der Copolymerisation vorgelegt oder vor oder während der Fällung des Copolymers zugegeben.

Vorzugsweise wird der bei der höheren Temperatur zerfallende Initiator zu Beginn der Copolymerisation vorgelegt oder vor der Fällung des Copolymers zugegeben.

Bei einer bevorzugten Initiatorkombination handelt es sich bei dem bei der niedrigeren Temperatur zerfallenden Initiator um Trigonox® EHP (Bis(2-ethylhexyl)peroxydicarbonat, CAS-Nr. 16111-62-9) und ist der bei der höheren Temperatur zerfallende Initiator ausgewählt unter tert.-Butylperoxypivalat (z. B. Luperox 11 M75 der Fa. Atochem), tert.-Butylperoctoat, Lauroylperoxid (LPO, CAS-Nr. 105-74-8) oder 2,5-Dimethyl-2,5-bis(t-butylperoxy)hexan (Trigonox® 101).

Eine weitere bevorzugte Initiatorkombination enthält Trigonox® EHP und tert.-Butylperoctoat.

Eine weitere bevorzugte Initiatorkombination enthält Lauroylperoxid und tert.-Butylperoctoat oder 2,5-Dimethyl-2,5-bis(t-butylperoxy)hexan (Tngonox® 101).

Eine weitere bevorzugte Initiatorkombination enthält tert.-Butylperoxypivalat (Luperox 11 M75 und tert.-Butylperoctoat oder 2,5-Dimethyl-2,5-bis(t-butylperoxy)hexan (Trigonox® 101).

Eine weitere bevorzugte Initiatorkombination enthält tert.-Butylperoctoat und 2,5-Dimethyl-2,5-bis(t-butylperoxy)hexan (Trigonox® 101).

Zur Erzielung möglichst reiner Polymere können die Polymere z. B. einem Waschschritt mit einem geeigneten Lösungsmittel, z. B. dem auch zur Polymerisation eingesetzten Lösungsmittel unterzogen werden.

Die Copolymere können durch verschiedene Trocknungsverfahren in eine feste Form, insbesondere ein Pulver, überführt werden. Durch Fällungspolymerisation erhaltene Polymere werden vor der Trocknung vorzugsweise durch Filtration isoliert. Bevorzugt werden Vakuumtrocknungsverfahren, wie die Vakuumwirbelbetttrocknung eingesetzt. Die so erhaltenen festen Copolymer-Zusammensetzungen lassen sich vorteilhafterweise erneut in wässrigen Medien lösen oder redispergieren. Geeignete wässrige Medien sind Wasser und Gemische aus Wasser mit wenigstens einem wassermischbaren Lösungsmittel. Bevorzugt sind Wasser und Wasser/Alkohol-Gemische, wobei der Alkohol vorzugsweise ausgewählt ist unter C₁-C₄-Alkanolen, wie Ethanol, Isopropanol, n-Butanol und tert.-Butanol. Feste Copolymere haben den Vorteil einer besseren Lagerfähigkeit, einer einfacheren Transportierbarkeit und zeigen in der Regel eine geringere Neigung für Keimbefall.

Die anionogenen Gruppen (Säuregruppen) der Copolymere können mit einer Base teilweise oder vollständig neutralisiert werden. Als Base für die Neutralisation der Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Für den Einsatz in Haarbehandlungsmitteln haben sich zur Neutralisation der Säuregruppen enthaltenden Polymere NaOH, KOH, 2-Amino-2-methyl-1-propanol, 2-Amino-2-ethylpropan-1,3-diol, Diethylaminopropylamin und Triisopropanolamin bewährt. Die Neutralisation der Säuregruppen kann auch mit Hilfe von Mischungen mehrerer Basen vorgenommen werden, z. B. Mischungen aus Natronlauge und Triisopropanolamin. Die Neutralisation kann je nach Anwendungszweck partiell oder vollständig erfolgen. Unter einer teilweisen Neutralisierung wird vorzugsweise eine Neutralisierung von bis zu 95 %, vorzugsweise bis zu 50 %, speziell von bis zu 25 % der im Polymer enthaltenen Säuregruppen verstanden. Unter einer teilweisen Neutralisierung wird des Weiteren vorzugsweise eine Neutralisierung von mindestens 1 %, vorzugsweise mindestens 5 % der im Polymer enthaltenen Säuregruppen verstanden.

Geladene kationische Gruppen lassen sich aus den vorliegenden kationogenen stickstoffhaltigen Gruppen entweder durch Protonierung, z. B. mit ein- oder mehrwertigen Carbonsäuren, wie Milchsäure oder Weinsäure, oder mit Mineralsäuren, wie Phosphorsäure, Schwefelsäure und Salzsäure, oder durch Quaternisierung, z. B. mit Alkylierungsmitteln, wie C₁-C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Sollen die efindungsgemäß verwendeten Copolymere sowohl quaternisiert als auch neutralisiert werden, so erfolgt vorzugsweise zuerst die Quatemisierung und anschließend die Neutralisierung.

Die zuvor beschriebenen Copolymere eignen sich in vorteilhafter Weise zur Modifizierung der rheologischen Eigenschaften von haarkosmetischen Mitteln. Insbesondere handelt es sich dabei um wässrige Zusammensetzungen. Vorteilhafterweise sind dabei die Zusammensetzungen der Copolymere im Allgemeinen klar. Somit können die haarkosmetischen Formulierungen ohne Beeinträchtigung durch die Eigenfarbe der Zusammensetzungen angefärbt werden. Des Weiteren können die Zusammensetzungen in Form von opaken bis klaren Gelen formuliert werden.

Die erfindungsgemäß verwendeten anionisch ampholytischen Copolymere eignen sich speziell als Rheologiemodifizierungsmittel mit über den pH-Wert steuerbaren Eigenschaften. Sie eignen sich speziell als pH-schaltbare Verdicker für einen pH-Bereich größer oder gleich 6.

Die erfindungsgemäß verwendeten quaternisierten kationischen Copolymere weisen eine hohe pH-Stabilität in einem pH-Bereich von etwa 3 bis 9 auf. Die erfindungsgemäß verwendeten (teil)neutralisierten Copolymere eignen sich speziell als Rheologiemodifizierungsmittel mit über den pH-Wert steuerbaren Eigenschaften. Sie eignen sich speziell als pH-schaltbare Verdicker für einen pH-Bereich von etwa 4 bis 7.

Die erfindungsgemäß verwendeten anionisch ampholytischen Copolymere und kationisch ampholytischen Copolymere eignen sich speziell auch als Rheologiemodifizierungsmittel für salzhaltige Zusammensetzungen. Andererseits lassen sich mit den erfindungsgemäßen Copolymeren auch salzfreie Zusammensetzungen verdicken.

Vorteilhafterweise wirken die erfindungsgemäß verwendeten anionisch ampholytischen Copolymere und kationisch ampholytischen Copolymere auch als filmbildende und/oder konditionierend wirkende Rheologiemodifizierungsmittel. Sie eignen sich somit speziell für Haarfestiger als "festigende Verdicker" und in Haarpflegemitteln als "konditionierende Verdicker".

Die Copolymere eignen sich sowohl zur Herstellung homogenphasiger wässriger Zusammensetzungen als auch zur Formulierung von heterogenphasigen Zusammensetzungen, die zusätzlich wenigstens eine wasserunlösliche (hydrophobe) flüssige oder feste Verbindung umfassen. "Homogenphasige Zusammensetzungen" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen fest/flüssig-, flüssig/flüssig- und fest/flüssig/flüssig-Zusammensetzungen, wie Dispersionen und Emulsionen, z. B. O/W - und W/O-Formulierungen, die wenigstens eine der im Folgenden näher beschriebenen Öl- bzw. Fettkomponenten und Wasser als nicht mischbare Phasen aufweisen. Prinzipiell können die Copolymere sowohl in der Wasserphase als auch in der Ölphase eingesetzt werden. Im Allgemeinen enthalten heterogenphasige flüssig/flüssig-Zusammensetzungen die Copolymere im Wesentlichen in der Wasserphase.

Ein weiterer Gegenstand der Erfindung ist ein haarkosmetisches Mittel, enthaltend
A) wenigstens ein ampholytisches Copolymer, wie zuvor definiert,
B) gegebenenfalls wenigstens ein von A) verschiedenes Haarpolymer,
C) wenigstens einen kosmetisch akzeptablen Träger, und
D) gegebenenfalls wenigstens einen von A) und B) verschiedenen kosmetisch akzeptablen Wirk- und/oder Hilfsstoff.

Die erfindungsgemäß verwendeten ampholytischen Copolymere A) zeichnen sich vorteilhafterweise nicht nur durch gute verdickende Eigenschaften, sondern auch durch filmbildende Eigenschaften aus. Sie können somit in den haarkosmetischen Mitteln auch als haarfestigende Komponente, eingesetzt werden, so dass der Einsatz zusätzlicher Festigerpolymere B) nur in verringerter Menge erforderlich ist oder sogar ganz überflüssig sein kann. Die ampholytischen Copolymere A) zeichnen sich vorteilhafterweise auch durch konditionierende Eigenschaften aus und können die sensorischen Eigenschaften des Haars verbessern, z. B. ihm Geschmeidigkeit und Glanz verleihen. Mit den Copolymeren A) behandelte Haare sind nicht oder nur sehr gering klebrig.

Die erfindungsgemäß verwendeten anionisch ampholytischen Copolymere A) zeichnen sich durch ihre guten Anwendungseigenschaften im pH-Bereich von 6 bis 10, vorzugsweise 6,5 bis 8 aus.

Die erfindungsgemäß verwendeten kationisch ampholytischen Copolymere A) zeichnen sich durch ihre guten Anwendungseigenschaften im pH-Bereich von 4 bis 9 aus.

Die kosmetischen Mittel enthalten die Polymerkomponente A) vorzugsweise in einem Anteil von etwa 0,1 bis 10 Gew.-%, besonders bevorzugt 0,2 bis 6 Gew.-%, insbesondere 0,3 bis 3 Gew.-%, bezogen auf das Gesamtgewicht des Mittels.

Geeignete Haarpolymere B) sind ganz allgemein filmbildende Polymere, die kosmetisch verträglich sind und zur Formulierung haarkosmetischer Zusammensetzungen Anwendung finden. Vorzugsweise handelt es sich um wasserlösliche oder wasserdispergierbare Polymere. Besonders bevorzugt sind wasserlösliche Haarpolymere. Dazu zählen ganz allgemein von den Copolymeren A) verschiedene anionische, kationische, amphotere und nichtionische Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, Polyasparaginsäure und deren Salze und Derivate, wasserlösliche oder wasserdispergierbare Polyester, Polyurethane, z. B. Luviset PUR® der Fa. BASF, und Polyhamstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z'. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Akylester der (Meth)acrylsäure, C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviftex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrytsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/-Vinylimidazoliumsalzen (Luviquat® FC, Luviquat® HM, Luviquat® MS, Luviset Clear ®, Luviquat Supreme ®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquatemium-4 und -10), Acrylamidocopolymere (Polyquatemium-7) und Chitosan. Geeignete kationische Polymere sind auch Polyethylenimine und deren Salze und Polyvinylamine und deren Salze. Geeignete kationische (quaternisierte) Polymere sind auch Merquat® (Polymer auf Basis von Dimethyldiallylammoniumchlorid), Gafquat® (quatemäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind nichtionische Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon sowie nichtionische Cellulosederivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyllnmethylammoniumchtorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersitoxane, wie Tegopren® (Fa. Goldschmidt) oder Belsil® (Fa. Wacker).

Die kosmetischen Mittel enthalten die Haarpolymere B) vorzugsweise in einem Anteil von etwa 0,001 bis 30 Gew.-%, besonders bevorzugt 0,01 bis 15 Gew.-%, insbesondere 0,1 bis 7 Gew.-%, bezogen auf das Gesamtgewicht des Mittels.

Vorzugsweise weisen die Zusammensetzungen eine Trägerkomponente C) auf, die ausgewählt ist unter Wasser, hydrophilen Komponenten, hydrophoben Komponenten und Mischungen davon.

Geeignete hydrophile Träger C) sind z. B. Wasser, ein-, zwei- oder mehrwertige Alkohole mit vorzugsweise 1 bis 8 Kohlenstoffatomen, wie Ethanol, n-Propanol, Isopropanol, Propylenglycol, Glycerin, Sorbit, etc. Bevorzugt als hydrophile Träger sind Wasser und Gemische aus Wasser und mindestens einem C₂-C₆-Alkanol, wie Ethanol, n-Propanol und Isopropanol.

Geeignete hydrophobe Träger C) sind vorzugsweise ausgewählt unter
i) Ölen, Fetten, Wachsen,
ii) von iii) verschiedenen Estern von C₆-C₃₀-Monocarbonsäuren mit ein-, zwei- oder dreiwertigen Alkoholen,
iii) gesättigten acyclischen und cyclischen Kohlenwasserstoffen,
iv) Fettsäuren,
v) Fettalkoholen,
vi) Treibgasen,
und Mischungen davon.

Geeignete Siliconöle C) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten C) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten C) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bevorzugte Träger C) sind weiterhin Treibmittel. Geeignete Treibmittel C) sind die für Haarsprays oder Aerosolschäume üblicherweise verwendeten. Bevorzugt sind Gemische aus Propan/Butan, Pentan, Dimethylether, 1,1-Difluorethan (HFC-152 a), Kohlendioxid, Stickstoff oder Druckluft.

Zusätzlich können die erfindungsgemäßen Mittel als Komponente D) wenigstens einen weiteren, von A) und B) verschiedenen kosmetischen Wirk- oder Hilfsstoff enthalten. Die Komponenten D) werden nach dem gewünschten Einsatzbereich der Zusammensetzung ausgewählt. Neben einsatzbereichtypischen Komponenten sind sie vorzugsweise ausgewählt unter kosmetisch aktiven Wirkstoffen, Exzipienten, Emulgatoren Tensiden, Konservierungsmitteln, Duftstoffen (z. B. Parfümölen), von Komponente A) verschiedenen Verdickern, von Komponente A) verschiedenen Polymeren (z. B. Haarpolymeren, Haarconditionern, Pfropfpolymeren, wasserlöslichen oder dispergierbaren silikonhaltigen Polymeren), Gelbildnern, Farbstoffen, Pigmenten, Lichtschutzmitteln, Konsistenzgebern, Antioxidantien, Bleichmitteln, Pflegemitteln, Tönungsmitteln, Bräunungsmitteln, Feuchthaltemitteln, Rückfettern, Collagen, Eiweißhydrolysaten, Lipiden, Emollienzien, Weichmachern, Entschäumern, Antistatika, Harzen, Lösemitteln, Lösungsvemnittlern, Neutralisierungsmitteln, Stabilisatoren, Sterilantien, Treibmitteln, Trocknungsmitteln, Trübungsmitteln, etc.

Die Copolymere A) können gemeinsam mit konventionellen Verdickern D) eingesetzt werden. Geeignete konventionelle Verdickungsmittel sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Bevorzugte Verdicker sind Verbindungen, die der Formulierung ein pseudoplastisches Fließverhalten verleimen, d. h. hohe Viskosität im Ruhezustand und niedrige Viskosität im bewegten Zustand. Besonders bevorzugt sind Xanthan Gum^{®} (Kelzan^{®} der Fa. Kelco), Rhodopol^{®} 23 (Rhone Poulenc), Veegum^{®} (Firma R. T. Vanderbilt) oder Attaday^{®} (Firma Engelhardt).

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrosphären eingesetzt werden.

Aufgrund ihrer verdickenden und filmbildenden Eigenschaften eignen sich die zuvor beschriebenen Copolymere A) insbesondere als Zusatzstoffe für Formulierung in Form von Gelen.

Bevorzugte kosmetisch aktive Wirkstoffe sind z. B. Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, desodorierende Wirkstoffe, sebostatische Wirkstoffe, Pflanzenextrakte, antierythimatös oder antiallergisch aktive Wirkstoffe und Mischungen davon.

Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Hamstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind die zuvor genannten. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heubluinenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Vorzugsweise liegen die efindungsgemäßen Haarbehandlungsmittel in Form eines Schaumfestigers, Haarmousses, Haargels, Haarwachses, Shampoos, Haarsprays, Haarschaums, Spitzenfluids, Egalisierungsmittels für Dauerwellen oder "Hot-Oil-Treatments", insbesondere in Form eines Haargels, Haarwachses oder Shampoos vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
- 0,05 bis 5 Gew.-% wenigstens eines Copolymers A),
- 0 bis 7 Gew.-%, z.B. 0,01 bis 5 Gew.-% wenigstens eines Haarfestigerpolymers B),
- 83 bis 99,95 Gew.-% wenigstens eines Trägers, ausgewählt unter Wasser und Wasser/Alkohol-Gemischen, die bis zu 20 Gew.-% Alkohol, bezogen auf das Gewicht des Wasser/Alkohol-Gemischs enthalten, sowie Treibmitteln,
- 0 bis 5 Gew.-%, vorzugsweise 0,01 bis 3 Gew.-% wenigstens eines weiteren Bestandteils.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Als Emulgatoren können alle in Haarschäume üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Ceteth-1, Polyethylenglykolcetylether; Cetearethe, z. B. Ceteareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimoniumbromid, Cocotrimoniummethylsulfat, Quatemium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder PropylenoxidEinheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 5 Gew.-% wenigstens eines Copolymers A),
b) 0 bis 5 Gew.-% wenigstens eines von A) verschiedenen kosmetisch akzeptablen wasserlöslichen oder wasserdispergierbaren Haarfestigerpolymers B),
c) 80 bis 99,85 Gew.-% Wasser und/oder Alkohol,
d) 0 bis 1 Gew.-% eines von A) verschiedenen Gelbildners,
e) 0 bis 20 Gew.-% weitere Bestandteile.

Als zusätzliche Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquatemium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquatemium 37 (und) Propylenglycoldicapratdicaprylat (und) PPG-1 Trideceth-6, Polyquatemium-7, Polyquaternium-44. Als zusätzliche Gelbildner geeignete vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat Polymere, wie Carbopol® Ultrez 21 von der Firma Noveon. Weitere Beispiele für als Gelbildner geeignete anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn® von der Firma Rohm und Haas erhältlich sind.

Die erfindungsgemäßen Copolymere A) können in haarkosmetischen Zubereitungen als Konditioniermittel eingesetzt werden.

Nach einer weiteren bevorzugten Ausführungsform handelt es sich bei den erfindungsgemäßen Mitteln um eine Shampoo-Formulierung.

Solche Formulierungen enthalten wenigstens ein Copolymer sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickem/Gelbildnem, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Shampoopräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Shampoos übliche kationische Tenside enthalten, wie z. B. quaternäre Ammoniumverbindungen, beispielsweise Cetyltrimethylammoniumchlorid.

Weiterhin können die Shampoo-Formulierungen Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

In den Shampooformulierungen können zur Erzielung bestimmter Effekte übliche Konditioniermittel in Kombination mit den Copolymeren A) eingesetzt werden. Hierzu zählen beispielsweise die zuvor genannten kationischen Polymere mit der Bezeichnung Polyquatemium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazofiumsalzen (Luviquat® Hold); kationische Cellulosederivate (Polyquaternium-4 und -10), Acrylamidcopolymere (Polyquatemium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die erfindungsgemäß verwendeten Copolymere eignen sich vorzugsweise für Shampoos, die keine oder nur geringe Anteile an anorganischen Salzen, speziell Alkali- und Erdalkalichloride, wie NaCl, enthalten. Sie eignen sich somit vorteilhaft zur Herstellung von milden Shampoos und Babyshampoos.

Ein weiterer Gegenstand der Erfindung ist Verfahren zur kosmetischen Behandlung der Haare, bei dem man ein haarkosmetisches Mittel, wie zuvor definiert, bereitstellt und, gegebenenfalls unter Zuhilfenahme der Hände, von Kämmen, Bürsten etc. und/oder von Wasser sowie gegebenenfalls unter anschließender Einwirkung von warmer Luft auf die zu behandelnden Haare appliziert. In einer speziellen Ausführung dieses Verfahrens werden die Haare nach der Applikation und gegebenenfalls einer Wirkzeit einem Auswaschvorgang unterzogen (z. B. bei Mitteln in Form eines Shampoos). In einer zweiten Ausführung verbleiben die Mittel nach der Applikation solange auf dem Haar, wie der mit ihnen verbundene haarkosmetische Effekt anhält oder erwünscht ist und werden erst dann durch Auswaschen entfernt.

Die Erfindung wird anhand der folgenden nicht einschränkenden Beispiele näher erläutert.

Allgemeine Herstellungsvorschrift A1 (anionisch ampholytische Copolymere):

### Beispiel 23: Copolymer aus VP/MAS/VI/MMA/C₁₆₋₁₈-PEG-MA/EGDMA

| | | |
|---|---|---|
| Vorlage: | 412 g | Butylacetat |
| | | |
| Zulauf 1: | 58,5 g | Vinylpyrrolidon |
| | 9,0 g | Vinylimidazol |
| | 66,0 g | Methaorytsäure |
| | 15,0 g | Plex-6877 O® (25%ige C₁₆-C₁₈-PEG-MA in Methylmethacrylat) |
| | 1,5 g | Ethylenglykoldimethacrylat |
| | | |
| Zulauf 2: | 38,2 g | Butylacetat |
| | 0,15 g | tert.-Buhrlperoctoat |
| | | |
| Zulauf 3: | 95,6 g | Butylacetat |
| | 0,39 g | tert.-Butylperoctoat |
| | | |
| Zulauf 4: | 15 g | Methylchlorid (ca. 1,5-fache des Gewichts von Vinylimidazol) |
| Zulauf 5: | 23 g | Triethanolamin (ca. 20 % bezogen auf Methacrylsäure) |

In einer Rührapparatur mit Rückflusskühler, Innenthermometer und vier Zulaufvorrichtungen wurden bei 85 bis 88 °C Zulauf 1 und Zulauf 2 in zwei Stunden zugegeben. Danach wurde das Reaktionsgemisch bei ca. 88 °C 2 h weiter gerührt. Anschließend wurde Zulauf 3 in 30 Minuten zudosiert und 3 Stunden bei 90 °C nachpolymerisiert. Das als weißes Pulver ausgefallene Produkt wurde mit Methylchlorid (Zulauf 4) bei einer Temperatur zwischen 70 und 100 °C quaternisiert. Nach dem Abkühlen auf ca. 40 °C wurde das Produkt mit Triethanolamin (Zulauf 5) in 1 h bei 40 °C teilneutralisiert. Das Pulver wurde über eine Filternutsche abgesaugt, mindestens zweimal mit Aceton gewaschen und bei 40 °C im Vakuum getrocknet.

Zur Herstellung der Polymere 26 bis 40 legte man Belsil® DMC 6031, Fa. Wacker, in der Vorlage vor.

In analoger Weise wurden die in Tabelle I angegebenen Polymere 2, 5, 7, 9, 13 bis 17, 20, 24, 25, 26, 31, 32, 34, 35, 36, 38, 39 und 40 hergestellt.

Die Polymere 3, 4, 6, 8, 10, 11, 12, 27 bis 30 und 33 wurden analog zur Herstellungsvorschrift A hergestellt, abgesehen davon, dass die Teilneutralisierung mit Triethanolamin entfiel.

Die Polymere 1, 18, 19, 21, 22 und 37 wurden analog zur Herstellungsvorschrift A1 hergestellt, abgesehen davon, dass die Quatemisierung mit Methylchlorid entfiel.

Tabelle I:VP/MAS/VI/MMA/C₁₈₋C₁₈-PEGMA/EGDMA-Fällungspolymerisate

| Beispiel Nr. | VP [Gew.-%] | VCap [Gew.-%] | MAS [Gew.-%] | VI [Gew.-%] | DMAEMA [Gew.-0/ol | SMA [Gew.-%] | MMA^{#} [Gew.-%] | C₁₆₋₁₈₋ PEG-MA^{#} [Gew.-%] | ODVE [Gew.-%] | EGDMA [Gew.-%] | Belsil® 6031 **) | Quatemisierung mit CH₃-Cl | Neutralisierung mit TEA, NG % |
|---|---|---|---|---|---|---|---|---|---|---|---|---|---|
| 1 | 40 | -- | 54 | 5 | -- | -- | -- | -- | -- | 1,0 | -- | -- | 20 |
| 2 | 43,7 | -- | 45 | 10 | -- | -- | -- | -- | -- | 1,3 | -- | ≥ 70% | 20 |
| 3 | 63,7 | -- | 30 | 5 | -- | -- | -- | -- | -- | 1,3 | -- | ≥ 70% | -- |
| 4 | 73,7 | -- | 20 | 5 | -- | -- | -- | -- | -- | 1,3 | -- | ≥ 70% | -- |
| 5 | 33,6 | 10 | 45 | 10 | -- | -- | -- | -- | -- | 1,4 | -- | ≥ 70% *) | 20 |
| 6 | 23,6 | 10 | 50 | 15 | -- | -- | -- | -- | -- | 1,4 | -- | ≥ 70% | -- |
| 7 | 43,8 | 10 | 35 | 10 | -- | -- | -- | -- | -- | 1,2 | -- | ≥ 70% *) | 20 |
| 8 | 53,8 | 10 | 27 | 8 | -- | -- | -- | -- | -- | 1,2 | -- | ≥ 70% | -- |
| 9 | 43,7 | -- | 45 | 10 | -- | -- | -- | -- | -- | 1,3 | -- | ≥ 70% *) | 20 |
| 10 | 33,6 | -- | 50 | 15 | -- | -- | -- | -- | -- | 1,4 | -- | ≥ 70% | -- |
| 11 | 18,5 | -- | 60 | 20 | -- | -- | -- | -- | -- | 1,5 | -- | ≥ 70% | -- |
| 12 | 30 | -- | 58,8 | 10 | -- | -- | -- | -- | -- | 1,2 | -- | ≥ 70% | -- |
| 13 | 30 | -- | 56 | 10 | -- | 2,8 | -- | -- | -- | 1,2 | -- | ≥ 70% *) | 20 |
| 14 | 44 | -- | 43 | 10 | -- | 2 | -- | -- | -- | 1,0 | -- | ≥ 70% *) | 20 |
| 15 | 40 | -- | 43 | 13 | -- | 3 | -- | -- | -- | 1,0 | -- | ≥ 70% *) | 20 |
| 16 | 38,7 | -- | 45 | 10 | -- | -- | 5 | -- | -- | 1.3 | -- | ≥ 70% *) | 20 |
| 17 | 33,7 | -- | 45 | 10 | -- | -- | 10 | -- | -- | 1,3 | -- | ≥ 70% *) | 20 |
| 18 | 39 | -- | 51,8 | 3 | -- | -- | 5 | -- | -- | 1,2 | -- | -- | 20 |
| 19 | 39 | -- | 45 | 5 | -- | -- | 10 | -- | -- | 1,0 | -- | -- | 20 |
| 20 | 28 | -- | 45 | 10 | -- | -- | 15,7 | -- | -- | 1,3 | -- | ≥ 70% *) | 20 |
| 21 | 35 | -- | 54 | 5 | -- | -- | 3,75 | 1,25 | -- | 1,0 | -- | -- | 20 |
| 22 | 40 | -- | 51 | 3 | -- | -- | 3,75 | 1,25 | -- | 1,0 | -- | - | 20 |
| 23 | 39 | -- | 44 | 6 | -- | -- | 7,5 | 2,5 | -- | 1,0 | -- | ≥ 70% *) | 20 |
| 24 | 39 | -- | 46 | 10 | -- | -- | -- | -- | 4 | 1,0 | -- | ≥ 70% *) | 20 |
| 25 | 40 | -- | 42 | 14 | -- | -- | -- | -- | 3 | 1,0 | -- | ≥ 70% *) | 20 |
| 26 | 43,7 | -- | 45 | 10 | -- | -- | -- | -- | -- | 1,3 | 1 | ≥ 70% *) | 20 |
| 27 | 33,6 | -- | 50 | 15 | -- | -- | -- | -- | -- | 1,4 | 0,5 | ≥ 70% | -- |
| 28 | 18,5 | -- | 60 | 20 | -- | -- | -- | -- | -- | 1,5 | 2 | ≥ 70% | -- |
| 29 | 30 | -- | 58.8 | 10 | -- | -- | -- | -- | -- | 1,2 | 1 | ≥ 70% | -- |
| 30 | 23,6 | 10 | 50 | 15 | -- | -- | -- | -- | -- | 1,4 | 1 | ≥ 70% | -- |
| 31 | 33,6 | 10 | 45 | 10 | -- | -- | -- | -- | -- | 1,4 | 1 | ≥ 70% *) | 20 |
| 32 | 40,8 | 10 | 40 | 8 | -- | -- | -- | -- | -- | 1,2 | 0.5 | ≥ 70% *) | 20 |
| 33 | 50,8 | 10 | 30 | 8 | -- | -- | -- | -- | -- | 1,2 | 0,5 | ≥ 70% | -- |
| 34 | 40 | -- | 43 | 13 | -- | 3 | -- | -- | -- | 1,0 | 0,5 | ≥ 70% *) | 20 |
| 35 | 38,7 | -- | 45 | 10 | -- | -- | 5 | -- | -- | 1,3 | 0,5 | ≥ 70% *) | 20 |
| 36 | 33,7 | -- | 45 | 10 | -- | -- | 10 | -- | -- | 1,3 | 1 | ≥ 70% *) | 20 |
| 37 | 39 | -- | 50 | 4,7 | -- | -- | 5 | -- | -- | 1,3 | 0,5 | -- | 20 |
| 38 | 33 | -- | 50 | 5,7 | -- | -- | 10 | -- | -- | 1,3 | 0,5 | ≥ 70% *) | 20 |
| 39 | 39 | -- | 44 | 6 | -- | -- | 7,5 | 2,5 | -- | 1,0 | 0,5 | ≥ 70% *) | 20 |
| 40 | | -- | 42 | 14 | -- | -- | -- | -- | 3 | 1,0 | 0,5 | ≥ 70% *) | 20 |

| | | | | | | | | | | | | | |
|---|---|---|---|---|---|---|---|---|---|---|---|---|---|
| VP = N-Vinylpyrrolidon VCap = N-Vinylcaprolactam MAS = Methacrylsäure VI = N-Vinylimidazol DMAEMA = N,N-Dimethylaminoethylmethacrylat SMA = Stearylmethacrylat MMA = Methylmethacrylat C₁₆-C₁₈-PEG-MA = mit einem C₁₆-C₁₈-Fettalkoholgemisch terminiertes Polyethylenglykolmethacrylat ODVE Octadecylvinylether EGDMA Ethylenglkoldimethacrylat TEA / NG% Triethanolamin / Neutralisationsgrad ^{*)}: Das Produkt wurde nach der Quatemisierung mit Triethanolamin teilneutralisiert. # MMA und C₁₆₋₁₈-PEGMA können gemeinsam, z. B. in Form des kommerziellen Produkts Plex-6877 O®, Firma Degussa, Deutschland eingesetzt werden. **) Belsil® 6031 = ethoxyliertes/propoxyliertes Dimethylsiloxan, Fa. Wacker, Deutschland, angegeben in %, bezogen auf 100 Gew.-% der gesamten Monomeren | | | | | | | | | | | | | |

Allgemeine Herstellungsvorschrift A2 (kationisch ampholytische Copolymere):

### Beispiel 61: Copolymer aus VP/MAS/VI/MMA/C₁₆-C₁₈- PEG-MA/ PETAE

| | | |
|---|---|---|
| Vorlage: | 412 g | Butylacetat |
| | | |
| Zulauf 1: | 37,5g | Vinylpyrrolidon |
| | 6 g | Methacrylsäure |
| | 75,75 g | Vinylimidazol |
| | 30,0 g | Plex® 6877-O (25%ige C₁₆-C₁₈-PEG-MA in Methylmethacrylat) |
| | 0,75 g | Pentaerythrittriallylether |
| | | |
| Zulauf 2: | 38,2 g | Butylacetat |
| | 0,15 g | tert.-Butylperoctoat |
| | | |
| Zulauf 3: | 95,6 g | Butylacetat |
| | 0,39 g | tert.-Butylperoctoat |
| | | |
| Zulauf 4: | 100 g | Methylchlorid |

In einer Druckapparatur mit Rührer, Rückflusskühler, Innenthermometer und vier separaten Zulaufvorrichtungen wurden bei 85 bis 88 °C Zulauf 1 und Zulauf 2 in zwei Stunden zugegeben. Das Reaktionsgemisch wurde bei ca. 88 °C noch 2 h gerührt. Danach wurde Zulauf 3 in 30 Minuten zudosiert und 3 Stunden bei 90 °C nachpolymerisiert. Das als weißes Pulver ausgefallene Produkt wurde mit Methylchlorid (Zulauf 4) in etwa 1 h bei ca. 90 °C quatemisiert. Das Pulver wurde über eine Filtemutsche abgesaugt, zweimal mit Aceton gewaschen und bei 40 °C im Vakuum getrocknet.

Nach dieser Vorschrift wurden die Polymere 41 bis 60 und 62 bis 93 der nachfolgenden Tabelle II hergestellt. In den Beispielen Nr. 74 bis 93 wurde Belsil® DMC 6031 in der Vorlage vorgelegt.

**Tabelle II: VI/MAS/VP-Fällungspolymerisate**

| Beispiel Nr. | VP [Gew.-%] | VCap [Gew.-%] | MAS [Gew,-%] | VI [Gew.-%] | DMAEMA [Gew.-%] | SMA [Gew.-%] | MMA [Gew.-%) | C₁₆-C₁₈PEG-MA [Gew.-%] | ODVE [Gew.-%] | PETAE [Gew.-%] | Belsil® DMC 6031 [Gew.-%] | Quatemisierung mit CH₃-Cl |
|---|---|---|---|---|---|---|---|---|---|---|---|---|
| 41 | 29,5 | -- | 2 | 68 | -- | -- | -- | -- | -- | 0,5 | -- | ≥ 70% |
| 42 | 79,5 | -- | 4 | 16 | -- | -- | -- | -- | -- | 0,5 | -- | ≥ 70% |
| 43 | 71 | -- | 3,5 | 25 | -- | -- | -- | -- | -- | 0,5 | -- | ≥ 70% |
| 44 | 66,5 | -- | 3 | 30 | -- | -- | -- | -- | -- | 0,5 | -- | ≥ 70% |
| 45 | 56,5 | -- | 3 | 40 | -- | -- | -- | -- | -- | 0,5 | -- | ≥ 70% |
| 46 | 46,5 | -- | 3 | 50 | -- | -- | -- | -- | -- | 0,5 | -- | ≥ 70% |
| 47 | 60 | 11,5 | 3 | 25 | -- | -- | -- | -- | -- | 0,5 | -- | ≥ 70% |
| 48 | 56 | 15 | 3,5 | 25 | -- | -- | -- | -- | -- | 0,5 | -- | ≥ 80% |
| 49 | 56,5 | 10 | 3 | 30 | -- | -- | -- | -- | -- | 0,5 | -- | ≥ 70% |
| 50 | 46 | 20 | 3,5 | 26 | -- | -- | -- | -- | -- | 0,5 | -- | ≥ 70% |
| 51 | 27 | -- | 3 | 67 | -- | 2,5 | -- | -- | -- | 0,5 | -- | ≥ 70% |
| 52 | 70 | -- | 3 | 25 | -- | 1,5 | -- | -- | -- | 0,5 | -- | ≥ 70% |
| 53 | 59 | -- | 4 | 30 | -- | 1,5 | -- | -- | -- | 0,5 | -- | ≥ 70% |
| 54 | 58 | -- | 4 | 35 | -- | 2,5 | -- | -- | -- | 0,5 | -- | ≥ 70% |
| 55 | 79,6 | -- | 3 | 10 | -- | -- | 7 | -- | -- | 0,4 | -- | ≥ 80% |
| 56 | 25 | -- | 3 | 62 | -- | -- | 9,5 | -- | -- | 0,5 | -- | ≥ 70% |
| 57 | 38 | -- | 3,5 | 43 | -- | -- | 15 | -- | -- | 0,5 | -- | ≥ 70% |
| 58 | 61,5 | -- | 4 | 25 | -- | -- | 10 | -- | -- | 0,5 | -- | ≥ 70% |
| 59 | 46 | -- | 3,5 | 30 | -- | -- | 20 | -- | -- | 0,5 | -- | ≥ 70% |
| 60 | 25 | -- | 3 | 61,5 | -- | -- | 7,5 | 2,5 | -- | 0.5 | -- | ≥ 70% |
| 61 | 25 | -- | 4 | 50,5 | -- | -- | 15 | 5 | -- | 0,5 | -- | ≥ 70% |
| 62 | 46,7 | -- | 4 | 34 | -- | -- | 7,5 | 2,5 | -- | 0,3 | -- | ≥ 70% |
| 63 | 56,5 | -- | 4 | 29 | -- | -- | 7.5 | 2,5 | -- | 0,5 | -- | ≥ 70% |
| 64 | 61,5 | -- | 3 | 25 | -- | -- | 7,5 | 2,5 | -- | 0,5 | -- | ≥ 70% |
| 65 | 66,5 | -- | 3 | 25 | -- | -- | 3,75 | 1,25 | -- | 0,5 | -- | ≥ 70% |
| 66 | 72 | -- | 3,5 | 20 | -- | -- | 3 | 1 | -- | 0,5 | -- | ≥ 80% |
| 67 | 79,6 | -- | 3 | 12 | -- | -- | 3,75 | 1,25 | -- | 0,5 | -- | ≥ 80% |
| 68 | 74,5 | -- | 4 | 16 | -- | -- | 3,75 | 1,25 | -- | 0,5 | -- | ≥ 70% |
| 69 | 55 | 10 | 3 | 30 | -- | -- | | -- | 1,5 | 0,5 | -- | ≥ 70% |
| 70 | 68 | -- | 4 | 26 | -- | -- | -- | -- | 1,5 | 0,5 | -- | ≥ 70% |
| 71 a) | 65 | -- | 4 | 16 | 10 | 4,5 | -- | -- | -- | 0.5 | -- | ≥ 70% |
| 72 a) | 65 | -- | 5 | 10 | 10 | -- | 9,5 | -- | -- | 0.5 | -- | ≥ 70% |
| 73 a) | 64,5 | -- | 5 | 20 | 5 | -- | 3,75 | 1,25 | -- | 0,5 | -- | ≥ 70% |
| 74 | 71 | -- | 3,5 | 25 | -- | -- | -- | -- | -- | 0,5 | 0,5 | ≥ 70% |
| 75 | 66,5 | -- | 3 | 30 | -- | -- | -- | -- | -- | 0,5 | 1 | ≥ 70% |
| 76 | 56,5 | -- | 3 | 40 | -- | -- | -- | -- | -- | 0,5 | 2 | ≥ 70% |
| 77 | 60 | 11,5 | 3 | 25 | -- | -- | -- | -- | -- | 0,5 | 0,5 | ≥ 70% |
| 78 | 56 | 15 | 3,5 | 25 | -- | -- | -- | -- | -- | 0,5 | 0,5 | ≥ 80% |
| 79 | 56,5 | 10 | 3 | 30 | -- | -- | -- | -- | -- | 0,5 | 1 | ≥ 70% |
| 80 | 46 | 20 | 3,5 | 26 | -- | -- | -- | -- | -- | 0,5 | 1 | ≥ 70% |
| 81 | 70 | -- | 3 | 25 | -- | 1,5 | -- | -- | -- | 0,5 | 0,5 | ≥ 70% |
| 82 | 59 | -- | 4 | 30 | -- | 1,5 | -- | -- | -- | 0,5 | 0,5 | ≥ 70% |
| 83 | 58 | -- | 4 | 35 | -- | 2,5 | -- | -- | -- | 0,5 | 0,5 | ≥ 70% |
| 84 | 61,5 | -- | 4 | 25 | -- | -- | 10 | -- | -- | 0,5 | 1 | ≥ 70% |
| 85 | 46 | -- | 3,5 | 30 | -- | -- | 20 | -- | -- | 0,5 | 1 | ≥ 70% |
| 86 | 46,7 | -- | 4 | 34 | -- | -- | 7,5 | 2,5 | -- | 0,3 | 1 | ≥ 70% |
| 87 | 56,5 | -- | 4 | 29 | -- | -- | 7,5 | 2,5 | -- | 0,5 | 1 | ≥ 70% |
| 88 | 66,5 | -- | 3 | 25 | -- | -- | 3,75 | 1,25 | -- | 0,5 | 1 | ≥ 70% |
| 89 | 72 | -- | 3,5 | 20 | -- | -- | 3 | 1 | -- | 0,5 | 0,5 | ≥ 80% |
| 90 | 55 | 10 | 3 | 30 | -- | -- | -- | -- | 1,5 | 0,5 | 2 | ≥ 70% |
| 91 | 68 | -- | 4 | 26 | -- | -- | -- | -- | 1,5 | 0,5 | 1 | ≥ 70% |
| 92 a) | 65 | -- | 5 | 10 | 10 | -- | 9,5 | -- | -- | 0,5 | 1 | ≥ 70% |
| 93 a) | 64,5 | -- | 5 | 20 | 5 | -- | 3,75 | 1,25 | -- | 0,5 | 1 | ≥ 70% |

| | | | | | | | | | | | | |
|---|---|---|---|---|---|---|---|---|---|---|---|---|
| a) nicht enfindungsgemäß VP = N-Vinylpyrrolidon VCap = N-Vinylcaprolactam MAS = Methacrylsäure VI = N-Vinylimidazol a) = nicht erfindungsgemäß DMAEMA = N,N-Dimethylaminoethylmethacrylat SMA = Stearylmethacrylat MMA = Methylmethacrylat C₁₆-C₁₈-PEG-MA = mit einem C₁₆-C₁₈-Fettalkoholgemisch terminiertes Polyethylenglykolmethacrylat ODVE Octadecylvinylether PETAE Pentaerythrittrialllylether MMA und C₁₆-C₁₈-PEG-MA können auch gemeinsam, z. B. in Form des kommerziellen Produkts Plex® 6877-O, Firma Degussa, Deutschland eingesetzt werden. Belsil® DMC 6031: ethoxyliertes/propoxyliertes Dimethylsiloxan, Fa. Wacker, Deutschland. | | | | | | | | | | | | |

Beispiel 114 (Variante B): Polymerisation von VI/MAS/VP/PETAE in Gegenwart eines ethoxilierten Dimethylsiloxans unter Verwendung von zwei Radikalinitiatoren mit unterschiedlicher Zerfallstemperatur

| | | |
|---|---|---|
| Vorlage: | 613 g | Butylacetat |
| | 2 g | Belsil® DMC 6031 |
| | 1 g | Trigonox® 101 (2,5-Dimethyl-2,5-di(tert.-butylperoxy)hexan) |
| | | |
| Zulauf 1: | 94 g | Vinylpyrrolidon |
| | 102 g | Vinylimidazol |
| | 6,95 g | Methacrylsäure |
| | 1,2 g | Pentaerythrittriallylether |
| | | |
| Zulauf 2: | 35 g | n-Butylacetat |
| | 0,2 g | tert.-Butylperoctoat |
| | | |
| Zulauf 3 | 175 | g n-butylacetat |
| | 1,0 g | tert.-Butylperoctoat |
| | | |
| Zulauf 4: | 175 g | n-butylacetat |
| | 1,0 g | tert.-Butylperoctoat |

In einer Apparatur, die mit Rührer, Rückflusszähler, Innenthermometer und vier Zulaufvorrichtungen ausgestattet war, erwärmte man die Vorlage unter Stickstoffatmosphäre auf 90°C. Die Zuläufe 1 und 2 wurden innerhalb von 3 h zugegeben und bei 90 °C weitere 1,5 h gerührt. Zulauf 3 wurde bei 100 °C in 1h zugeben, der Reaktionsansatz 1 h bei dieser Temperatur gerührt. Anschließend wurde Zulauf 4 bei 100 °C innerhalb von 1 h zugegeben und dann nochmals 2 h bei 100 °C gerührt. Die Temperatur wurde auf 125 °C erhöht und bei dieser Temperatur noch 2 h weitergerührt. Die erhaltene weiße Suspension wurde anschließend mit 50 g Methylchlorid quatemisiert. Das Produkt wurde filtriert, mit Aceton gewaschen und bei 70 °C im Vakuum getrocknet.

In analoger Weise wurden alle Produkte - der Variante B gemäß Tabelle III hergestellt.

Beispiel 96 (Variante C): Polymerisation von AS/DMAPMAM/SMA/PETAE in Gegenwart eines ethoxilierten Dimethylsiloxans unter Verwendung von zwei Radikalinitiatoren mit unterschiedlicher Zerfallstemperatur.

| | | |
|---|---|---|
| Vorlage: | 800 g | Ethylacetat |
| | 1,6 g | Belsil® DMC 6031 |
| | 1 g | tert.-Butylperoctoat |
| | | |
| Zulauf 1: | 164 g | Acrylsäure |
| | | |
| Zulauf 2: | 7,8 g | DMAPMAM |
| | 1,3 g | Pentaerythrittriallylether |
| | 3,5 g | Stearylmethacrylat |
| | | |
| Zulauf 3 | 80 g | Ethylacetat |
| | 0,4 g | Lauroylperoxid |
| | | |
| Zulauf 4: | 200 g | Ethylacetat |
| | 0,4 g | Lauroylperoxid |

In einer Apparatur, die mit Rührer, Rückflusszähler, Innenthermometer und vier Zulaufvorrichtungen ausgestattet war, erwärmte man die Vorlage unter Stickstoffatmosphäre und Rühren auf 75 °C. Die Zuläufe 1, 2 und 3 wurden innerhalb von 3 h zugegeben und bei 75 °C weitere 2 h gerührt. Zulauf 4 wurde in 1 h bei 80 °C zugeben und dann noch 1 h weitergerührt. Die Temperatur wurde auf 100 °C erhöht und bei dieser Temperatur noch weitere 3 h gerührt. Die erhaltene weiße Suspension wurde anschließend mit 40 g Methylchlorid quatemisiert. Das Produkt wurde filtriert, mit Aceton gewaschen und bei 70 °C im Vakuum getrocknet.

In analoger Weise wurden alle Produkte der Variante C gemäß Tabelle III hergestellt.

Beispiel 94 (Variante D): Polymerisation von AS/DMAPMAM/SMA/PETAE in Gegenwart eines ethoxilierten Dimethylsiloxans unter Verwendung von zwei Radikalinitiatoren mit unterschiedlicher Zerfallstemperatur

| | | |
|---|---|---|
| Vorlage: | 670 g | Ethylacetat/Cyclohexan (65:35) |
| | 2 g | Belsil® DMC 6031 |
| | 50 g | Zulauf 1 |
| | 14 g | Zulauf 2 |
| | 1,5 g | Pentaerythrittriallylether |
| | 1,5 g | tert.-Butylperoctoat |
| | | |
| Zulauf 1: | 142,5g | Acrylsäure |
| | 3 g | Stearylmethacrytat |
| | 3 g | Dimethylaminopropylmethacrylamid |
| | 100 g | Ethylacetat/Cyclohexan (65:35) |
| | 4,3 g | wasserfreies K₂CO₃ |
| | | |
| Zulauf 2: | 70 g | Ethylacetat/Cyclohexan (65:35) |
| | 0,35 g | Trigonox® EHP-C75 (75%ig) |
| | | |
| Zulauf 3 | 70 g | Ethylacetat/Cyclohexan (65:35) |
| | 1,0 g | Trigonox® EHP-C75 (75%ig) |

In einer Apparatur, die mit Rührer, Rückflusszähler, Innenthermometer und drei Zulaufvorrichtungen ausgestattet war, erwärmte man die Vorlage unter Stickstoffatmosphäre und Rühren auf 50 °C. Der Zulauf 1 wurde innerhalb von 1,5 h und der Zulauf 2 innerhalb von 2 h zugegeben und der Ansatz bei 60 °C noch weitere 2 h gerührt. Zulauf 3 wurde in 1 h bei 60 °C zugegeben und dann noch 2 h bei 70 °C gerührt. Die Temperatur wurde auf 100 °C erhöht und bei der Temperatur noch 3 h weitergerührt. Die erhaltene weiße Suspension wurde filtriert, mit Aceton gewaschen und bei 70 °C im Vakuum getrocknet.

In analoger Weise wurden alle Produkte der Variante D gemäß Tabelle III hergestellt.

**Tabelle III:**

| Bsp. | (EO)-Silicon^{#} | VP^{#} | MAS^{#} | AS^{#} | VI^{#} | DMAPMAM^{#} | SMA^{#} | EGDMA^{#} | PETAE^{#} | Herstellung-variante |
|---|---|---|---|---|---|---|---|---|---|---|
| 94 a) | 1 | | | 95 | | 2,0 | 2,0 | | 1,0 | D |
| 95 a) | 1 | | | 92 | | 2,0 | 5,0 | | 1,0 | D |
| 96 a) | 1 | | | 92 | | 5,0 | 2,0 | | 1,0 | C |
| 97 a) | 1 | | 90 | | | 8,5 | | 1,5 | | B |
| 98 a) | 1 | | 90 | | | 5,0 | 3,5 | 1,5 | | B |
| 99 a) | 1 | 23,8 | 70 | | | 5,0 | | 1,2 | | B |
| 100 a) | 1 | 45,8 | 50 | | | 3,0 | | 1,2 | | B |
| 101 a) | 1 | 45,8 | | 50 | | 3,0 | | | 1,2 | D |
| 102 a) | 1 | 45,0 | | 48 | | 3,0 | 2,8 | | 1,2 | D |
| 103 a) | | | | 95 | | 2,0 | 2,0 | | 1,0 | D |
| 104 a) | | | | 92 | | 2,0 | 5,0 | | 1,0 | D |
| 105 a) | | | | 92 | | 5,0 | 2,0 | | 1,0 | C |
| 106 a) | | | 90 | | | 8,5 | | 1,5 | | B |
| 107 a) | | | 90 | | | 5,0 | 3.5 | 1,5 | | B |
| 108 a) | | 23,8 | 70 | | | 5,0 | | 1,2 | | B |
| 109 a) | | 45,8 | 50 | | | 3,0 | | 1,2 | | B |
| 110 a) | | 45,8 | | 50 | | 3,0 | | | 1,2 | D |
| 111 a) | | 45,0 | | 48 | | 3,0 | 2,8 | | 1,2 | D |
| 112 | 1 | 70,0 | 3,4 | | 26 | | | | 0,6 | B* |
| 113 | 1 | 60,0 | 3,4 | | 36 | | | | 0,6 | B |
| 114 | 1 | 46,0 | 3,4 | | 50 | | | | 0,6 | B |
| 115 | 1 | 16,0 | 3,4 | | 80 | | | | 0,6 | B |
| 116 | 1 | | 3,4 | | 95 | | | | 0,6 | B |
| 117 | 1 | | 3,0 | | 94 | | | | 0,5 | B |
| 118 | | 70,0 | 3,4 | | 26 | | | | 0,6 | B* |
| 119 | | 60,0 | 3,4 | | 36 | | | | 0,6 | B |
| 120 | | 46,0 | 3,4 | | 50 | | | | 0,6 | B |
| 121 | | 16,0 | 3,43 | | 80 | | | | 0,6 | B |
| 122 | | | 3,4 | | 95 | | | | 0,6 | B |
| 123 | | | 3,0 | | 94 | | | | 0,5 | B |

| | | | | | | | | | | |
|---|---|---|---|---|---|---|---|---|---|---|
| a) nicht erfindungsgemäß Bsp. Beispiel ^{#} Die Mengenangaben sind in Gew.%, bezogen auf die zur Polymerisation eingesetzten ungesättigten Verbindungen, die Gewichtsteile Si-haltiger Verbindung sind separat angegeben (EO)-Silicon Belsil® 6031, ethoxiliertes Dimethylsiloxan VP Vinylpyrrolidon MAS Methacrylsäure AS Acrylsäure VI Vinylimidazol DMAPMAM Dimethylaminopropylmethacrylamid SMA Stearylmethacrylat EGDMA Ethylenglykoldimethacrylat PETAE Pentaerythrittriallylether EMA Ethylmethacrylat n-BA n-Butylacrylat PLEX-O Plex® 6877-0 = Methacrylsäureester eines mit 25 Mol Ethylenoxid alkoxilierten C₁₆-C₁₈-Fettalkohols als 25%ige Lösung in Methylmethacrylat * zu 70 % mit CH₃Cl quaternisiert | | | | | | | | | | |

### II. Anwendungstechnische Beispiele

Anwendungstechnische Beispiele 1 - 58:

Haargele mit einem nichtionischen Haarfestiger:

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/ 97/ 98/ 99/ 100/ 101/ 102/ 103/ 104/ 105/ 106/ 107/ 108/ 109/ 110/ 111 | | 2,0 |
| Luviskol® K90 | Polyvinylpyrrolidon | 1,5 |
| Wasser, dest. | | 46,5 |
| Weiterer Zusatz: Konservierungsmittel, z. B. Euxyl® K100, Parfüm, usw. | | |
| mit Triethanolamin (50%ige Lösung) auf pH zwischen 6,7 und 7,2 einstellen | | |

| Phase 2: | | |
|---|---|---|
| Wasser, dest. | | ad 100 |

### Herstellung:

Phase 1 wurde eingewogen und unter Rühren bei einer Temperatur im Bereich von 20 bis 50 °C homogenisiert. Nach etwa 3 Stunden entstand eine milchige Dispersion. Man gab Triethanolamin unter Rühren zu. Nach etwa 2 Stunden wurde ein (fast) homogenes, hoch viskoses Gel gebildet. Danach rührte man Phase 2 langsam in Phase 1 ein. Das Gel wurde bei Raumtemperatur noch 1 Stunde gerührt. Dabei entstand ein stabiles, klares Gel.

In gleicher Weise wurde ein Standardgel (Vergleichsgel VG) mit Carbopol® 940 und Luviskol® K90, hergestellt, wobei Phase 1 anstelle von 2,0 Gew.-% des Polymers aus dem Beispiel Nr. 1 und 1,5 Gew.-% Luviskol K90 0,5 Gew.-% Carbopol® 940 und 3 Gew.-% Luviskol® K90 enthielt.

Haargele der Anwendungsbeispiele 5, 16, 30 beziehungsweise 35, die das Polymer aus den Beispielen Nr. 5, 16, 30 beziehungsweise 35 jeweils in einer Menge von 2,0 Gew.-% sowie Luviskol® K90 jeweils in einer Menge von 1,5 Gew.-% enthielten, wurden auf ihre anwendungstechnischen Eigenschaften (Klarheit, Klebrigkeit, Nasskämmbarkeit und Festigung) untersucht. Als Vergleich diente das zuvor beschriebene Vergleichsgel VG. Der Polymergehalt sowohl der erfindungsgemäßen Gele, wie auch des Vergleichsgels, betrug somit 3,5 %.

Dem Test lag die folgende Bewertungsskala zugrunde:

| | Klarheit | Klebrigkeit | Nasskämmbarkeit | Festigung |
|---|---|---|---|---|
| Note 1 | klar | nicht klebrig | sehr gut | sehr gut |
| Note 2 | leicht trüb | leicht klebrig | gut | gut |
| Note 3 | trüb | klebrig | mäßig | noch gut |

| | Klarheit | Klebrigkeit | Nasskämm-barkeit | Festigung |
|---|---|---|---|---|
| Vergleichsgel, VG | 1-2 | 2-3 | 2-3 | 1-2 |
| Gel mit Polymer aus Bsp. Nr. 5 | 1 | 2 | 1-2 | 1 |
| Gel mit Polymer aus Bsp. Nr. 16 | 1 | 1-2 | 2 | 1 |
| Gel mit Polymer aus Bsp. Nr. 30 | 1 | 1-2 | 1-2 | 1 |
| Gel mit Polymer aus Bsp. Nr. 35 | 1-2 | 1-2 | 2 | 1 |

Anwendungstechnische Beispiele 59 - 123:

Haargele mit einem nichtionischen Haarfestiger:

| | CTFA | Gew.-% |
|---|---|---|
| Phase 1: | | |
| Polymer (kationisch ampholytisch) aus Beispiel Nr. 41 / 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91/ 92/ 93/ 112/ 113/ 114/ 115/ 116/ 117/ 118/ 119/ 120/ 121/ 123 | | 2,0 |
| Luviskol® K90 | Polyvinylpyrrolidon | 1,5 |
| Wasser, dest. | | 46,5 |

| Phase 2: | | |
|---|---|---|
| Wasser dest. | | ad 100,0 |
| Weiterer Zusatz: Konservierungsmittel, z. B. Euxyl® K100, Parfüm, usw. | | |

### Herstellung:

Phase 1 wurde eingewogen und unter Rühren bei einer Temperatur im Bereich von 20 bis 50 °C homogenisiert. Nach etwa 2 Stunden bildete sich ein (fast) homogenes, festes Gel. Danach rührte man Phase 2 langsam in Phase 1 ein. Das Gel wurde bei Raumtemperatur noch 1 Stunde gerührt. Dabei entstand ein stabiles, klares Gel.

In gleicher Weise wurde ein Standardgel (Vergleichsgel VG) mit Carbopol® 940 und Luviskol® K90 hergestellt, wobei Phase 1 anstelle von 2,0 Gew.-% des Polymers aus dem Beispiel Nr. 1 und 1,5 Gew.-% Luviskol® K90 0,5 Gew.-% Carbopol® 940 und 3 Gew.-% Luviskol® K90 enthielt.

Haargele der Anwendungsbeispiele 50, 51, 58, 80, 81 beziehungsweise 86, die das Polymer aus den Beispielen Nr. 50, 51, 58, 80, 81 beziehungsweise 86 jeweils in einer Menge von 2,0 Gew.-% sowie Luviskol® K90 jeweils in einer Menge von 1,5 Gew.-% enthielten, wurden auf ihre anwendungstechnischen Eigenschaften (Klarheit, Klebrigkeit, Nasskämmbarkeit und Festigung) untersucht. Als Vergleich diente das zuvor beschriebene Vergleichsgel VG. Der Polymergehalt sowohl der erfindungsgemäßen Gele, wie auch des Vergleichsgels betrug somit 3,5 %.

Dem Test lag die obige Bewertungsskala zugrunde.

Ergebnisse des Tests:

| | Klarheit | Klebrigkeit | Nasskämm-barkeit | Festigung |
|---|---|---|---|---|
| Vergleichsgel VG | 1-2 | 2-3 | 2-3 | 1-2 |
| Gel mit Polymer aus Bsp. Nr. 50 | 1 | 1-2 | 1-2 | 1 |
| Gel mit Polymer aus Bsp. Nr. 51 | 1-2 | 1-2 | 1-2 | 1 |
| Gel mit Polymer aus Bsp. Nr. 58 | 1 | 1-2 | 1-2 | 1 |
| Gel mit Polymer aus Bsp. Nr. 80 | 1 | 1-2 | 1-2 | 1 |
| Gel mit Polymer aus Bsp. Nr. 81 | 1 | 1-2 | 1-2 | 1 |
| Gel mit Polymer aus Bsp. Nr. 86 | 1 | 1-2 | 1-2 | 1-2 |

| | | | | |
|---|---|---|---|---|
| Bsp. Beispiel | | | | |

Anwendungstechnische Beispiele 124-181:

Haargele mit einem nichtionischen Haarfestiger:

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/ 97/ 98/ 99/ 100/ 101/ 102/ 103/ 104/ 105/ 106/ 107/ 108/ 109/ 110/ 111 | | 1,8 |
| Luviskol® K90 | Polyvinylpyrrolidon | 1,0 |
| Wasser, dest | | 46,8 |
| Weiterer Zusatz: Konservierungsmittel, z. B. Euxyl® K100, Parfüm, usw. | | |
| mit Triethanolamin (50%ige Lösung) auf pH zwischen 6,7 und 7,2 einstellen | | |

| Phase 2: | | |
|---|---|---|
| Wasser, dest | | ad 100 |

### Herstellung:

Phase 1 wurde eingewogen und unter Rühren bei einer Temperatur im Bereich von 20 bis 50 °C homogenisiert. Nach etwa 3 Stunden entstand eine milchige Dispersion. Man gab Triethanolamin unter Rühren zu. Nach etwa 2 Stunden wurde ein (fast) homogenes, hoch viskoses Gel gebildet Danach rührte man Phase 2 langsam in Phase 1 ein. Das Gel wurde bei Raumtemperatur noch 1 Stunde gerührt. Dabei entstand ein stabiles, klares Gel.

Haargele der Anwendungsbeispiele 128, 139, 153 beziehungsweise 158, die das Polymer aus den Beispielen Nr. 5, 16, 30 beziehungsweise 35 jeweils in einer Menge von 1,8 Gew.-% sowie Luviskol® K90 jeweils in einer Menge von 1,0 Gew.-% enthielten, wurden auf ihre anwendungstechnischen Eigenschaften untersucht. Als Vergleichsgel diente das zuvor beschriebene. Der Polymergehalt der erfindungsgemäßen Gele betrug somit nur 2,8 %, der des Vergleichsgels 3,5 %. Dem Test lag die obige Bewertungsskala zugrunde:

| | Klarheit | Klebrigkeit | Nasskämm-barkeit | Festigung |
|---|---|---|---|---|
| Vergleichsgel, VG | 1-2 | 2-3 | 2-3 | 1-2 |
| Gel mit Polymer aus Bsp. Nr. 5 | 1 | 2 | 2 | 1-2 |
| Gel mit Polymer aus Bsp. Nr. 16 | 1 | 1-2 | 2 | 1-2 |
| Gel mit Polymer aus Bsp. Nr. 30 | 1 | 1-2 | 1-2 | 1-2 |
| Gel mit Polymer aus Bsp. Nr. 35 | 1-2 | 1-2 | 2 | 1-2 |

Wie der Tabelle zu entnehmen ist, werden mit den erfindungsgemäßen Gelen selbst bei verringertem Polymergehalt bessere Formulierungen erzielt als mit dem Vergleichsgel.

Anwendungstechnische Beispiele 182 - 246:

Haargele mit einem nichtionischen Haarfestiger:

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91 / 92/ 53/ 112/ 113/ 114/ 115/ 116/ 117/ 118/ 119/ 120/ 121/ 122/ 123 | | 2,0 |
| Luviskol® K90 | Polyvinylpyrrolidon | 0,7 |
| Wasser, dest. | | 46,8 |
| Weiterer Zusatz: Konservierungsmittel, z. B. Euxyl® K100, Parfüm, usw. | | |
| Mit Triethanolamin (50%ige Lösung) auf pH 6,7- 7,2 einstellen. | | |

| Phase 2: | | |
|---|---|---|
| Wasser dest. | | ad 100,0 |

### Herstellung:

Phase 1 wurde eingewogen und unter Rühren bei einer Temperatur im Bereich von 20 bis 50 °C homogenisiert. Nach etwa 2 Stunden bildete sich ein (fast) homogenes, festes Gel. Danach rührte man Phase 2 langsam in Phase 1 ein. Das Gel wurde bei Raumtemperatur noch 1 Stunde gerührt. Dabei entstand ein stabiles, klares Gel.

Haargele der Anwendungsbeispiele 191, 192, 199, 221, 222 beziehungsweise 227, die das Polymer aus den Beispielen Nr. 50, 51, 58, 80, 81 beziehungsweise 86 jeweils in einer Menge von 2,0 Gew.-% sowie Luviskol® K90 jeweils in einer Menge von 0,7 Gew.-% enthielten, wurden auf ihre anwendungstechnischen Eigenschaften untersucht. Als Vergleichsgel diente das zuvor beschriebene. Der Polymergehalt der erfindungsgemäßen Gele betrug somit nur 2,7 %, der des Vergleichsgels 3,5 %. Dem Test lag die obige Bewertungsskala zugrunde:

Ergebnisse des Tests:

| | Klarheit | Klebrigkeit | Nasskämm-barkeit | Festigung |
|---|---|---|---|---|
| Vergleichsgel VG | 1-2 | 2-3 | 2-3 | 1-2 |
| Gel mit Polymer aus Bsp. Nr. 50 | 1 | 1-2 | 1-2 | 1 |
| Gel mit Polymer aus Bsp. Nr. 51 | 1-2 | 1-2 | 1-2 | 1-2 |
| Gel mit Polymer aus Bsp. Nr. 58 | 1 | 1-2 | 1-2 | 1 |
| Gel mit Polymer aus Bsp. Nr. 80 | 1 | 1-2 | 1-2 | 1 |
| Gel mit Polymer aus Bsp. Nr. 81 | 1-2 | 1-2 | 1-2 | 1 |
| Gel mit Polymer aus Bsp. Nr. 86 | 1-2 | 1-2 | 1-2 | 1-2 |

Wie der Tabelle zu entnehmen ist, werden mit den erfindungsgemäßen Gelen selbst bei verringertem Polymergehalt bessere Formulierungen erzielt als mit dem Vergleichsgel.

Anwendungstechnische Beispiele 247 - 304:

Haargele mit Poly(Vinylpyrrolidon/Vinylacetat)

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/ 97/ 98/ 99/ 100/ 101/ 102/ 103/ 104/ 105/ 106/ 107/ 108/ 109/ 110/ 111 | | 2,0 |
| Luviskol® VA64 | Poly(Vinylpyrroiidon/Vinylacetat) | 1,5 |
| Wasser, dest. | | 46,5 |
| Weiterer Zusatz: Konservierungsmittel, z. B. Euxyl® K100, Parfüm usw. | | |
| mit Triethanolamin (50%ige Lösung) auf pH zwischen 6,7 - 7,2 einstellen. | | |

| Phase 2: | | |
|---|---|---|
| Wasser, dest. | | ad 100 |

### Herstellung:

Phase 1 wurde eingewogen und unter Rühren bei einer Temperatur im Bereich von 20 bis 50 °C homogenisiert. Nach etwa 3 Stunden entstand eine milchige Dispersion. Man gab Triethanolamin unter Rühren zu. Nach etwa 2 Stunden wurde ein (fast) homogenes, hoch viskoses Gel gebildet. Danach rührte man Phase 2 langsam in Phase 1 ein. Das Gel wurde bei Raumtemperatur noch 1 Stunde gerührt. Dabei entstand ein stabiles, fast klares bis klares Gel.

Anwendungstechnische Beispiele 305 - 369:

Haargele mit Poly(Vinylpyrrolidon/Vinylactat):

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91/ 92/ 53/ 112/ 113/ 114/ 115/ 116/ 117/ 118/ 119/ 120/ 121/ 122/ 123 | | 2,0 |
| Luviskol® VA64 | Poly(Vinylpyrrolidon/Vinylacetat) | 1,5 |
| Wasser, dest. | | 46,5 |

| Phase 2: | | |
|---|---|---|
| Wasser, dest. | | ad 100 |
| Weiterer Zusatz: Konservierungsmittel, z. B. Euxyl® K100, Parfüm, usw. | | |

### Herstellung:

Phase 1 wurde eingewogen und unter Rühren bei einer Temperatur im Bereich von 20 bis 50 °C homogenisiert. Nach etwa 2 Stunden bildete sich ein (fast) homogenes, festes Gel. Danach rührte man Phase 2 langsam in Phase 1 ein. Das Gel wurde bei Raumtemperatur noch 1 Stunde gerührt. Dabei entstand ein stabiles, fast klares bis klares Gel.

Anwendungstechnische Beispiele 370 - 427:

Haargele mit einem kationischen Haarpolymer

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ | | 2,2 |
| 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/ 97/ 98/ 99/ 100/ 101/ 102/ 103/ 104/ 105/ 106/ 107/ 108/ 109/ 110/ 111 | | |
| Luviskol® K90 | Polyvinylpyrrolidon | 0,8 |
| Luviquat® Supreme (BASF AG) | Polyquaternium-68 | 0,5 |
| Wasser, dest. | | 46,5 |
| Weiterer Zusatz: Konservierungsmittel, z.B. Euxyl® K100, Parfüm, usw. | | |
| mit Triethanolamin (50%ige Lösung) auf pH zwischen 6,7- 7,2 einstellen | | |

| Phase 2: | | |
|---|---|---|
| Wasser, dest. | | ad 100 |

### Herstellung:

Phase 1 wurde eingewogen und unter Rühren bei einer Temperatur im Bereich von 20 bis 50 °C homogenisiert. Nach etwa 3 Stunden entstand eine milchige Dispersion. Man gab Triethanolamin unter Rühren zu. Nach etwa 2 Stunden wurde ein (fast) homogenes, hoch viskoses Gel gebildet. Danach rührte man Phase 2 langsam in Phase 1 ein. Das Gel wurde bei Raumtemperatur noch 1 Stunde gerührt. Dabei entstand ein stabiles, fast klares bis klares Gel.

Anwendungstechnische Beispiele 428 - 492:

Haargele mit einem kationischen Haarpolymer

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91/ 92/ 53/ 112/ 113/ 114/ 115/ 116/ 117/ 118/ 119/ 120/ 121/ 122/ 123 | | 2,2 |
| Luviskol® K90 | Polyvinylpyrrolidon | 0,8 |
| Luviset® Clear (Fa. BASF) | Poly(VP/Methacrylamid/VI) | 0,5 |
| Wasser, dest. | | 46,5 |
| | | |

| Phase 2: | | |
|---|---|---|
| Wasser dest. | | ad 100,0 |
| Weiterer Zusatz: Konservierungsmittel, z. B. Euxyl® K100, Parfüm, usw. | | |

### Herstellung:

Phase 1 wurde eingewogen und unter Rühren bei einer Temperatur im Bereich von 20 bis 50 °C homogenisiert. Nach etwa 2 Stunden bildete sich ein (fast) homogenes, festes Gel. Danach rührte man Phase 2 langsam in Phase 1 ein. Das Gel wurde bei Raumtemperatur noch 1 Stunde gerührt. Dabei entstand ein stabiles, fast klares bis klares Gel.

Anwendungstechnische Beispiele 493 - 550:

Flexible Haarfestigergele

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/97/ 98/ 99/ 100/ 101/ 102/ 103/ 104/ 105/ 106/ 107/ 108/ 109/ 110/ 111 | | 2,0 |
| Luviskol® K90 | Polyvinylpyrrolidon | 1,0 |
| D-Panthenol, USP | Panthenol | 0,2 |
| Glycerin | | 0,1 |
| Wasser, dest. | | 46,7 |
| Weiterer Zusatz: Konservierungsmittel, z. B. Euxyl® K100, Parfüm, usw. | | |
| mit Triethanolamin (50%ige Lösung) auf pH zwischen 6,7 - 7,2 einstellen | | |

| Phase 2: | | |
|---|---|---|
| Wasser, dest. | | ad 100 |

### Herstellung:

Phase 1 wurde eingewogen und unter Rühren bei einer Temperatur im Bereich von 20 bis 50 °C homogenisiert. Nach etwa 3 Stunden entstand eine milchige Dispersion. Man gab Triethanolamin unter Rühren zu. Nach etwa 2 Stunden wurde ein (fast) homogenes, hoch viskoses Gel gebildet. Danach rührte man Phase 2 langsam in Phase 1 ein. Das Gel wurde bei Raumtemperatur noch 1 Stunde gerührt. Dabei entstand ein stabiles, fast klares bis klares Gel.

Anwendungstechnische Beispiele 551 - 615:

Flexible Haarfestigergele

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91/ 92/ 53/ 112/ 113/ 114/ 115/ 116/ 117/ 118 /119/ 120/ 121/ 122/ 123 | | 2,0 |
| Luviskol® K90 | Polyvinylpyrrolidon | 1,0 |
| D-Panthenol, USP | Panthenol | 0,2 |
| Glycerin | | 0,1 |
| Wasser, dest | | 46,7 |

| Phase 2: | | |
|---|---|---|
| Wasser, dest. | | ad 100,0 |
| Weiterer Zusatz: Konservierungsmittel, z. B. Euxyl® K100, Parfüm, usw. | | |

### Herstellung:

Phase 1 wurde eingewogen und unter Rühren bei einer Temperatur im Bereich von 20 bis 50 °C homogenisiert. Nach etwa 2 Stunden bildete sich ein (fast) homogenes, festes Gel. Danach rührte man Phase 2 langsam in Phase 1 ein. Das Gel wurde bei Raumtemperatur noch 1 h gerührt. Dabei entstand ein stabiles, fast klares bis klares Gel.

Anwendungstechnische Beispiele 616 - 673:

### Schaumfestiger

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/ 97/ 98/ 99/ 100/ 101/ 102/ 103/ 104/ 105/ 106/ 107/ 108/ 109/ 110/ 111 | | 0,5 |
| Wasser | | 28,0 |
| mit Triethanolamin (50%ig) auf pH 6,8 einstellen | | |

| Phase 2: | | |
|---|---|---|
| Luviset® Clear | | 1,2 |
| Wasser | | 60,0 |
| Cremophor® A 25 | Ceteareth 25/ BASF | 0,2 |
| Comperlan® KD | Coamide DEA/Henkel | 0,1 |
| Weitere Zusatz: Parfüm, Konservierungsmittel, usw. | | |
| Dimethylether | | 10,0 |

### Herstellung :

Phase 1: Polymer in Wasser einrühren. Das Polymer wurde unter Rühren mit Triethanolamin neutralisiert und gelöst. Phase 2 in Phase 1 langsam einrühren. Abfüllen und Treibgas zusetzen.

Anwendungstechnische Beispiele 674 - 738:

### Schaumfestiger

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91/ 92/ 53/ 112/ 113/ 114/ 115/ 116/ 117/ 118/ 119/ 120/ 121/ 122/ 123 | | 0,5 |
| Wasser | | 28,0 |

| Phase 2: | | |
|---|---|---|
| Luviquat® Supreme (Fa. BASF) | P(VP/MAM/VI/QVI) | 1,2 |
| Wasser | | 60,0 |
| Cremophor® A 25 | Ceteareth 25 (Fa. BASF) | 0,2 |
| Comperlan® KD | Coamide DEA (Fa. Henkel) | 0,1 |
| Weiterer Zusatz: Parfüm, Konservierungsmittel, usw. | | |
| Dimethylether | | 10,0 |

### Herstellung:

Phase 1: Polymer in Wasser einrühren. Danach wurde unter Rühren mit Triethanolamin neutralisiert und gelöst. Anschließend rührte man Phase 2 langsam in Phase 1 ein. Man füllte den Schaumfestiger ab und gab Treibgas zu.

Anwendungstechnische Beispiele 739 - 796:

### Schaumfestiger

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33/ 34/ 35/ 36/ 37/ 38/ 39/ 40/ 94/ 95/ 96/ 97/ 98/ 99/ 100/ 101/ 102/ 103/ 104/ 105/ 106/ 107/ 108/ 109/ 110/ 111 | | 0,8 |
| Wasser | | 37,7 |
| mit Triethanolamin (50%ig) auf pH 6,8 einstellen | | |

| Phase 2: | | |
|---|---|---|
| Luviskol® VA 64 | | 1,2 |
| Cremophor® A 25 | Ceteareth 25/ BASF | 0,2 |
| Comperlan® KD | Coamide DEA / Henkel | 0,1 |
| Wasser | | 50,0 |
| Weiterer Zusatz: Parfüm, Konservierungsmittel, usw. | | |
| Dimethylether | | 10,0 |

### Herstellung :

Phase 1: Polymer in Wasser einrühren. Das Polymer wurde unter Rühren mit Triethanolamin neutralisiert und gelöst. Phase 2 in Phase 1 langsam einrühren. Abfüllen und Treibgas zusetzen.

Anwendungstechnische Beispiele 797 - 861:

### Schaumfestiger

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91 / 92/ 53/ 112/ 113/ 114/ 115/ 116/ 117/ 118/ 119/ 120/ 121/ 122/ 123 | | 0,7 |
| Wasser | | 37,7 |

| Phase 2: | | |
|---|---|---|
| Luviskol® VA 64 | Poly(Vinylpyrolidon/Vinylacetat) | 1,0 |
| Luviskol® K12 | | 0,3 |
| Cremophor® A 25 | Ceteareth 25 | 0,2 |
| Comperlan® KD | Coamide DEA | 0,1 |
| Wasser | | 50,0 |
| Weiterer Zusatz: Parfüm, Konservierungsmittel, usw. | | |
| Dimethylether | | 10,0 |

### Herstellung:

Phase 1: Polymer in Wasser einrühren. Das Polymer wurde unter Rühren mit Triethanolamin neutralisiert und gelöst. Danach rührte man Phase 2 langsam in Phase 1 ein. Zuletzt füllte man ab und gab Treibgas zu.

Anwendungstechnische Beispiele 862 - 919:

### Shampoo (ohne Salzzusatz)

| | CTFA | Gew.-% |
|---|---|---|
| Phase 1: | | |
| Polymer (anionisch ampholytisch) aus Beispiel Nr. 1/ 2/ 3/ 4/ 5/ 6/ 7/ 8/ 9/ 10/ 11/ 12/ 13/ 14/ 15/ 16/ 17/ 18/ 19/ 20/ 21/ 22/ 23/ 24/ 25/ 26/ 27/ 28/ 29/ 30/ 31/ 32/ 33 34/ 35/ 36/ 37/ 38/ 40/ 94 95/ 96/ 97/ 98/ 99/ 100/ 101/ 102/ 103/ 104/ 105/ 106/ 107/ 108/ 109/ 110/ 111 | | 1,0 |
| Wasser | | 48,0 |
| mit Triethanolamin (50%ig) auf pH 6,8 einstellen | | |

| Phase 2: | | |
|---|---|---|
| Texapon® NSO 28%ig | Sodium Laureth Sulphate / Henkel | 50,0 |
| Comperlan® KD | Coamide DEA / Henkel | 1,0 |
| Weiterer Zusatz: Parfüm, Konservierungsmittel, usw. | | |

### Herstellung:

Einwiegen und unter Rühren Phasen 1 und 2 getrennt lösen und mischen. Phase 2 langsam in Phase 1 einrühren.

Anwendungstechnische Beispiele 920 - 984:

### Shampoo

| | CTFA | Gew.-% |
|---|---|---|
| Phase 1: | | |
| Polymer (kationisch ampholytisch) aus Beispiel Nr. 41/ 42/ 43/ 44/ 45/ 46/ 47/ 48/ 49/ 50/ 51/ 52/ 53/ 54/ 55/ 56/ 57/ 58/ 59/ 60/ 61/ 62/ 63/ 64/ 65/ 66/ 67/ 68/ 69/ 70/ 71/ 72/ 73/ 74/ 75/ 76/ 77/ 78/ 79/ 80/ 81/ 82/ 83/ 84/ 85/ 86/ 87/ 88/ 89/ 90/ 91 / 92/ 53/ 112/ 113/ 114/ 115/ 116/ 117/ 118/ 119/ 120/ 121/ 122/ 123 | | 1,0 |
| Polyvinylpyrrolidon K30 | | 0,5 |
| Wasser | | 47,5 |

| Phase 2: | | |
|---|---|---|
| Texapon® NSO 28%ig | Sodium Laureth | 50,0 |
| | Sulphate (Fa. Henkel) | |
| Comperlan® KD | Coamide DEA | 1,0 |
| Weiterer Zusatz: q.s. Parfümöl, Konservierungsmittel | | |

### Herstellung:

Einwiegen und unter Rühren Phasen 1 und 2 getrennt lösen und mischen. Phase 2 langsam in Phase 1 einrühren.

## Patentansprüche

1. verwendung eines ampholytischen Copolymers, erhältlich durch radikalische Copolymerisation nach der Methode der Fällungspolymerisation, das einen molaren Überschuss an anionogenen/anionischen Gruppen gegenüber kationogenen/kationischen Gruppen aufweist oder das einen molaren Überschuss an kationogenen/kationischen Gruppen gegenüber anionogenen/anionischen Gruppen aufweist und das Verbindungen, bestehend aus
a1) 2 bis 96 Gew.-% Acrylsäure und/oder Methacrylsäure,
a2) 2 bis 96 Gew.-% wenigstens einer N-Vinylimidazol-Verbindung der allgemeinen Formel (II) 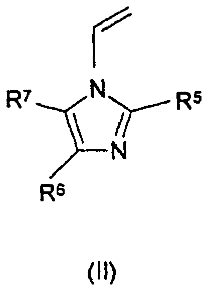 worin R⁵ bis R⁷ unabhängig voneinander für Wasserstoff, C₁-C₄-Alkyl oder Phenyl stehen, oder die durch Protonierung oder Quaternisierung der N-Vinylimidazol-Verdindungen erhältlichen Verbindungen,
b) 0,05 bis 5 Gew.-% Ethylenglykoldi(meth)acrylat und/oder Pentaerythrittriallylether,
c) 0 bis 15 Gew.-% wenigstens einer Siliconverbindung,
d) 0 bis 95 Gew.-% Vinylpyrrolidon und/oder Vinylcaprolactam,
e) 0 bis 40 Gew.-% wenigstens einer Verbindung, die ausgewählt ist unter C₈-C₂₂-(Meth)acrylaten, C₈-C₂₂-Alkylvinylethem, mit C₈-C₂₂-Alkylgruppen terminierten Polyether(meth)acrylaten, mit C₈-C₂₂-Alkylgruppen terminierten Allylalkoholalkoxilaten, C₈-C₂₂-Carbonsäurevinylestem und Mischungen davon,
f) 0 bis 40 Gew.-% wenigstens eines Monomers, das ausgewählt ist unter Methyl(meth)acrylat, Ethyl(meth)acrylat, n-Butyl(meth)acrylat und Mischungen davon,
einpolymerisiert enthält,
wobei das ampholytische Copolymer einen molaren Überschuss an anionogenen/anionischen Gruppen gegenüber kationogenen/kationischen Gruppen oder einen molaren Überschuss an kationogenen/kationischen Gruppen gegenüber anionogenen/anionischen Gruppen von wenigstens 2,5 : 1 aufweist,
als Rheologiemodifizierungsmittel für haarkosmetische Zusammensetzungen.

2. Verwendung nach Anspruch 1, wobei wenigstens ein Teil der Verbindungen a1) und a2) in Form eines Monomerenpaares eingesetzt wird, wobei das molare Verhältnis von anionogenen Gruppen der Komponente a1) zu kationogenen Gruppen der Komponente a2) 1 : 1 beträgt und wobei die im Unterschuss eingesetzte Komponente a1) oder a2) vollständig als Komponente des Monomerenpaares eingesetzt wird.

3. Verwendung nach einem der Ansprüche 1 oder 2, wobei die Komponente a2) 1-Vinylimidazol ist.

4. Verwendung nach einem der Ansprüche 1 bis 3 eines ampholytischen Copolymers, dessen kationogene Gruppen nach der Copolymerisation mit CH₃Cl oder Dimethylsulfat quaternisiert wurden.

5. Verwendung nach einem der vorhergehenden Ansprüche eines kationischen Silicongruppen-haltigen Copolymers, das einer teilweisen oder vollständigen Quaternisierung unterzogen wurde.

6. Verwendung nach einem der vorhergehenden Ansprüche eines ampholytischen Copolymers, dessen anionogene Gruppen nach der Copolymerisation mit einer Base, vorzugsweise einem Trialkanolamin, speziell Triethanolamin, teilneutralisiert wurden.

7. Verwendung nach einem der Ansprüche 1 bis 6, wobei das ampholytische Copolymer erhältlich ist durch radikalische Copolymerisation von
- wenigstens 2 Gew.-%, bezogen auf das Gesamtgewicht der zur Polymerisation eingesetzten Monomere, wenigstens eines Monomerenpaars aus N-Vinylimidazol und Acrylsäure und/oder Methacrylsäure,
- 5 bis 70 Gew.-% Methacrylsäure und/oder Acrylsäure,
- 0,1 bis 2 Gew.-% Ethylenglykoldi(meth)acrylat und/oder Pentaerythrittriallylether,
- 20 bis 95 Gew.-% Vinylpyrrolidon und/oder Vinylcaprolactam,
- 0 bis 40 Gew.-% wenigstens eines weiteren Monomers, das ausgewählt ist unter Methyl(meth)acrylat, Ethyl(meth)acrylat, n-Butyl(meth)acrylat, C₈-C₂₂-(Meth)acrylaten, mit C₈-C₂₂-Alkylgruppen terminierten Polyether(meth)acrylaten, C₈-C₂₂-Carbonsäurevinylestem und Mischungen davon.

8. Verwendung eines Copolymers wie in einem der Ansprüche 1 bis 7 definiert, das einen molaren Überschuss an kationogenen/kationischen Gruppen gegenüber anionogenen/anionischen Gruppen von wenigstens 6 : 1 aufweist.

9. Verwendung eines Copolymers, wie in einem der Ansprüche 1 bis 8 definiert, bei dem zur Copolymerisation zwei Initiatoren eingesetzt werden, deren Zerfallstemperaturen um wenigstens 10 °C, vorzugsweise um wenigstens 15 °C, voneinander verschieden sind.

10. Verwendung nach einem der Ansprüche 1 bis 8 zur Verbesserung der Festigungswirkung und/oder zur Verbesserung der Nasskämmbarkeit der haarkosmetischen Zusammensetzung.

11. Verfahren zur kosmetischen Behandlung der Haare, bei dem man ein haarkosmetisches Mittel, enthaltend
A) wenigstens ein ampholytisches Copolymer, wie in einem der Ansprüche 1 bis 8 definiert,
B) gegebenenfalls wenigstens ein von A) verschiedenes Haarpolymer,
C) wenigstens einen kosmetisch akzeptablen Träger, und
D) gegebenenfalls wenigstens einen von A) und B) verschiedenen kosmetisch akzeptablen Wirk- und/oder Hilfsstoff,
bereitstellt und auf die zu behandelnden Haare appliziert.

12. Verfahren nach Anspruch 11, wobei das Mittel in Form eines Schaumfestigers, Haarmousses, Haargels, Haarwachses, Shampoos, Haarsprays, Haarschaums, Spitzenfluids, Egalisierungsmittels für Dauerwellen oder Hot-Oil-Treatments, insbesondere in Form eines Haargels, Haarwachses oder Shampoos, vorliegt.

## Claims

1. The use of an ampholytic copolymer, obtainable by free-radical copolymerization in accordance with the method of precipitation polymerization, which has a molar excess of anionogenic/anionic groups compared with cationogenic/cationic groups or which has a molar excess of cationogenic/cationic groups compared with anionogenic/anionic groups and which comprises in copolymerized form, compounds consisting of
a1) 2 to 96% by weight of acrylic acid and/or methacrylic acid,
a2) 2 to 96% by weight of at least one N-vinylimidazole compound of the general formula (II) 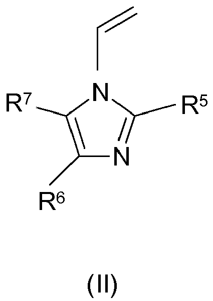 in which R⁵ to R⁷, independently of one another, are hydrogen, C₁-C₄-alkyl or phenyl, or the compounds obtainable by protonation or quarternization of the N-vinylimidazole compounds,
b) 0.05 to 5% by weight of ethylene glycol di(meth)acrylate and/or pentaerythritol triallyl ether,
c) 0 to 15% by weight of at least one silicone compound,
d) 0 to 95% by weight of vinylpyrrolidone and/or vinylcaprolactam,
e) 0 to 40% by weight of at least one compound which is chosen from C₈-C₂₂-(meth)acrylates, C₈-C₂₂-alkyl vinyl ethers, polyether (meth)acrylates terminated with C₈-C₂₂-alkyl groups, allyl alcohol alkoxylates terminated with C₈-C₂₂-alkyl groups, C₈-C₂₂-carboxylic acid vinyl esters and mixtures thereof,
f) 0 to 40% by weight of at least one monomer which is chosen from methyl (meth)acrylate, ethyl (meth)acrylate, n-butyl (meth)acrylate and mixtures thereof,
where the ampholytic copolymer has a molar excess of anionogenic/anionic groups compared with cationogenic/cationic groups or a molar excess of cationogenic/cationic groups compared with anionogenic/anionic groups of at least 2.5:1,
as rheology modifier for hair cosmetic compositions.

2. The use according to claim 1, where at least some of the compounds a1) and a2) are used in the form of a monomer pair, where the molar ratio of anionogenic groups of component a1) to cationogenic groups of component a2) is 1:1 and where the component a1) or a2) used in deficit is used completely as component of the monomer pair.

3. The use according to one of claims 1 or 2, where the component a2) is 1-vinylimidazole.

4. The use according to one of claims 1 to 3 of an ampholytic copolymer whose cationogenic groups after the copolymerization have been quarternized with CH₃Cl or dimethyl sulphate.

5. The use according to one of the preceding claims of a cationic silicone-group-containing copolymer which has been subjected to a partial or complete quarternization.

6. The use according to one of the preceding claims of an ampholytic copolymer whose anionogenic groups after the copolymerization have been partially neutralized with a base, preferably a trialkanolamine, specifically triethanolamine.

7. The use according to one of claims 1 to 6, where the ampholytic copolymer is obtainable by free-radical copolymerization of
- at least 2% by weight, based on the total weight of the monomers used for the polymerization, of at least one monomer pair of N-vinylimidazole and acrylic acid and/or methacrylic acid,
- 5 to 70% by weight of methacrylic acid and/or acrylic acid,
- 0.1 to 2% by weight of ethylene glycol di(meth)acrylate and/or pentaerythritol triallyl ether,
- 20 to 95% by weight of vinylpyrrolidone and/or vinylcaprolactam,
- 0 to 40% by weight of at least one further monomer which is chosen from methyl (meth)acrylate, ethyl (meth)acrylate, n-butyl (meth)acrylate, C₈-C₂₂-(meth)acrylates, polyether (meth)acrylates terminated with C₈-C₂₂-alkyl groups, C₈-C₂₂-carboxylic acid vinyl esters and mixtures thereof.

8. The use of a copolymer as defined in one of claims 1 to 7 which has a molar excess of cationogenic/cationic groups compared with anionogenic/anionic groups of at least 6:1.

9. The use of a copolymer, as defined in one of claims 1 to 8, in which, for the copolymerization, two initiators are used whose decomposition temperatures are different from one another by at least 10°C, preferably by at least 15°C.

10. The use according to one of claims 1 to 8 for improving the setting effect and/or for improving the wet combability of the hair cosmetic composition.

11. A method of cosmetically treating the hair in which a hair cosmetic composition comprising
A) at least one ampholytic copolymer as defined in one of claims 1 to 8,
B) optionally at least one hair polymer different from A),
C) at least one cosmetically acceptable carrier, and
D) optionally at least one cosmetically acceptable active ingredient and/or auxiliary different from A) and B),
is prepared and applied to the hair to be treated.

12. The method according to claim 11, where the composition is present in the form of a setting foam, hair mousse, hair gel, hair wax, shampoo, hair spray, hair foam, end fluid, neutralizer for permanent waves or hot-oil treatment, in particular in the form of a hair gel, hair wax or shampoo.

## Revendications

1. Utilisation d'un copolymère ampholyte, pouvant être obtenu par copolymérisation radicalaire selon la méthode de polymérisation par précipitation, qui présente un excès molaire de groupes anioniques/anionogènes par rapport aux groupes cationiques/cationogènes ou qui présente un excès molaire de groupes cationiques/cationogènes par rapport aux groupes anioniques/anionogènes et qui contient, incorporés par polymérisation, des composés, consistant en
a1) 2 à 96% en poids d'acide acrylique et/ou d'acide méthacrylique,
a2) 2 à 96 % en poids d'au moins un composé N-vinylimidazole de formule générale (II) 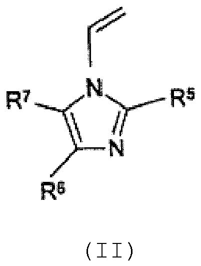 dans laquelle R⁵ à R⁷ représentent indépendamment l'un de l'autre un atome d'hydrogène, un groupe alkyle en C₁-C₄ ou phényle, ou des composés pouvant être obtenus par protonation ou quaternisation des composés N-vinylimidazole,
b) 0,05 à 5 % en poids de di(méth)acrylate d'éthylèneglycol et/ou d'éther triallylique de pentaérythritol,
c) 0 à 15 % en poids d'au moins un composé silicone,
d) 0 à 95 % en poids de vinylpyrrolidone et/ou vinylcaprolactame,
e) 0 à 40 % en poids d'au moins un composé qui est choisi parmi des (méth)acrylates en C₈-C₂₂, alkyl (C₈-C₂₂) vinyléthers, polyéther (méth) acrylates terminés par des groupes alkyle en C₈-C₂₂, produits d'alcoxylation d'alcool allylique terminés par des groupes alkyle en C₈-C₂₂, esters vinyliques d'acides carboxyliques en C₈-C₂₂ et des mélanges de ceux-ci,
f) 0 à 40 % en poids d'au moins un monomère qui est choisi parmi le (méth)acrylate de méthyle, le (méth)acrylate d'éthyle, le (méth)acrylate de n-butyle et des mélanges de ceux-ci,
le copolymère ampholyte présentant un excès molaire de groupes anioniques/anionogènes par rapport aux groupes cationiques/cationogènes ou un excès molaire de groupes cationiques/cationogènes par rapport aux groupes anioniques/anionogènes d'au moins 2,5:1,
en tant qu'agent modificateur de rhéologie pour compositions cosmétiques capillaires.

2. Utilisation selon la revendication 1, dans laquelle au moins une partie des composés a1) et a2) est utilisée sous forme d'une paire de monomères, dans laquelle le rapport molaire des groupes anionogènes du composant a1) aux groupes cationogènes du composant a2) est égal à 1 : 1 et dans laquelle le composant a1) ou a2) utilisé en quantité insuffisante est utilisé en totalité comme composant de la paire de monomères.

3. Utilisation selon la revendication 1 ou 2, dans laquelle le composant a2) est le 1-vinylimidazole.

4. Utilisation selon l'une quelconque des revendications 1 à 3 d'un copolymère ampholyte, dont les groupes cationogènes ont été quaternisés avec CH₃Cl ou du sulfate de diméthyle après la copolymérisation.

5. Utilisation selon l'une quelconque des revendications précédentes d'un copolymère contenant des groupes silicone cationiques, qui a été soumis à une quaternisation partielle ou totale.

6. Utilisation selon l'une quelconque des revendications précédentes d'un copolymère ampholyte, dont les groupes anionogènes ont été partiellement neutralisés après la copolymérisation par une base, de préférence une trialcanolamine, en particulier la triéthanolamine.

7. Utilisation selon l'une quelconque des revendications 1 à 6, dans laquelle le copolymère ampholyte peut être obtenu par copolymérisation radicalaire de
- au moins 2 % en poids, par rapport au poids total des monomères utilisés dans la polymérisation, d'au moins une paire de monomères constituée de N-vinylimidazole et acide acrylique et/ou acide méthacrylique,
- 5 à 70 % en poids d'acide méthacrylique et/ou d'acide acrylique,
- 0,1 à 2 % en poids de di(méth)acrylate d'éthylèneglycol et/ou d'éther triallylique de pentaérythritol,
- 20 à 95 % en poids de vinylpyrrolidone et/ou vinylcaprolactame,
- 0 à 40 % en poids d'au moins un autre monomère qui est choisi parmi le (méth)acrylate de méthyle, le (méth)acrylate d'éthyle, le (méth)acrylate de n-butyle, des (méth)acrylates en C₈-C₂₂, des polyéther(méth)acrylates terminés par des groupes alkyle en C₈-C₂₂, des esters vinyliques d'acides carboxyliques en C₈-C₂₂ et des mélanges de ceux-ci.

8. Utilisation d'un copolymère tel que défini dans l'une quelconque des revendications 1 à 7, qui présente un excès molaire de groupes cationiques/cationogènes, par rapport aux groupes anioniques/anionogènes, d'au moins 6 : 1.

9. Utilisation d'un copolymère, tel que défini dans l'une quelconque des revendications 1 à 8, dans lequel on utilise dans la copolymérisation deux amorceurs, sont les températures de décomposition diffèrent l'une de l'autre d'au moins 10 °C, de préférence d'au moins 15 °C.

10. Utilisation selon l'une quelconque des revendications 1 à 8, pour l'amélioration de l'effet fixateur et/ou pour l'amélioration de la facilité de passage du peigne à l'état humide de la composition cosmétique capillaire.

11. Procédé pour le traitement cosmétique des cheveux, dans lequel on fournit et applique sur les cheveux à traiter un produit cosmétique capillaire contenant
A) au moins un copolymère ampholyte, tel que défini dans l'une quelconque des revendications 1 à 8,
B) éventuellement au moins un polymère pour cheveux différent de A),
C) au moins un véhicule cosmétiquement acceptable, et
D) éventuellement au moins un principe actif et/ou adjuvant cosmétiquement acceptable, différent de A) et B).

12. Procédé selon la revendication 11, dans lequel le produit se trouve sous forme d'un fixateur en mousse, d'une mousse capillaire, d'un gel capillaire, d'une cire capillaire, d'un shampooing, d'une laque pour cheveux, d'une mousse pour cheveux, d'un liquide à pulvériser, d'un produit d'égalisation pour ondulations permanentes ou de Hot-Oil-Treatments, en particulier sous forme d'un gel capillaire, d'une cire capillaire ou d'un shampooing.
